# EUROPEAN PATENT SPECIFICATION

(11) **EP 1 296 625 B1**
(45) Date of publication and mention of the grant of the patent: **03.09.2008**
(21) Application number: 01935621.1
(22) Date of filing: 17.05.2001
(51) Int. Cl.: A61F 13/537

(54) **ABSORBENT ARTICLES HAVING IMPROVED PERFORMANCE**
ABSORBIERENDER ARTIKEL MIT VERBESSERTEM VERHALTEN
ARTICLES ABSORBANTS PRESENTANT UNE EFFICACITE AMELIOREE

(30) Priority: 17.05.2000 US 204643 P; 14.05.2001 US 855047
(43) Date of publication of application: 02.04.2003
(73) Proprietor: KIMBERLY-CLARK WORLDWIDE, INC., Neenah, WI 54956 (US)
(72) Inventor: WOON, Paul, S., 10110 Bangkok (TH); WOON, Lin-Sun, 10110 Bangkok (TH)
(74) Representative: Davies, Christopher Robert
(86) International application number: PCT/US2001/015925
(87) International publication number: WO 2001/087366

(56) References cited:
- WO-A-00/09056
- WO-A-00/71067
- GB-A- 2 272 859
- US-A- 5 334 177
- US-A- 5 348 547
- US-A- 5 364 382
- US-A- 5 624 423
- US-A- 5 785 697
- US-A- 5 792 129
- US-A- 5 853 402

## Description

### FIELD OF THE INVENTION

The present invention is generally in the field of absorbent articles. More specifically, the present invention relates to a personal care article having improved performance. The present invention is directed to personal care articles, including feminine pads having improved performance.

### BACKGROUND OF THE INVENTION

In the manufacture of absorbent articles, such as personal care articles, there is continual effort to improve the performance characteristics of the article. While these articles typically have many components, the in-use performance of some articles may be influenced primarily by components that distribute and/or absorb liquid. Accordingly, manufacturers strive to find ways of improving the liquid handling properties of materials used in these articles to improve performance.

One means of improving performance of these types of articles has been the extensive use of cellulose fluff and/or superabsorbent materials. For example, recent trends in commercial diaper designs have been to use more superabsorbent materials and less fiber to make the diaper thinner. However, notwithstanding the increase in total absorbent capacity contributed by the addition of larger amounts of superabsorbent material, such diapers often still suffer from excessive leaking during use.

One reason that absorbent articles with a high content of superabsorbent materials still leak is that many superabsorbent materials are unable to absorb liquid at the rate at which the liquid is applied to the absorbent composite during use.

The addition of fibrous material to the absorbent composite improves the leakage of an absorbent composite by temporarily holding the liquid until the superabsorbent material absorbs it. Fibers also serve to separate the particles of superabsorbent material to reduce the occurrence likelihood of gel-blocking. As used herein, the term "gel-blocking" refers to the situation wherein particles of superabsorbent material deform during swelling and block the interstitial spaces between the particles, or between the particles and the fibers, thus preventing the flow of liquid through the interstitial spaces. Even when fibrous material is incorporated into an absorbent composite, a poor choice of a superabsorbent material, especially one which exhibits gel-blocking behavior within the absorbent composite, results in poor liquid handling properties initially and later in the life cycle of the absorbent composite. Consequently, the choice of a particular superabsorbent material greatly affects the in-use absorbency and leakage of the absorbent product.

Another problem with some commercially available absorbent articles, such as diapers, is the tendency of the articles to leak after multiple insults. As used herein, the term "insults" refers to a single introduction of liquid into the absorbent material. For example, during use, a diaper is typically exposed to multiple insults during the life cycle of the diaper. To reduce diaper leakage during the life cycle of the diaper, it is desirable to maintain the level of intake performance of the absorbent composite throughout the life of the product.

Still another problem is that after the article has been subjected to an insult, the wearer of the article may feel wetness from liquid, even if the liquid has been absorbed by the article. In the case of blood and or urine, these liquids will irritate the skin of a person if they remain in contact with the person's skin. As such, if the absorbent article is unable to pull the liquid away from the surface of the article that contacts the wearer, the wearer's skin may become irritated, even if the article does not leak.

Lastly, in the case of some absorbent articles, such as feminine pads, the absorbent materials used may be able to absorb the liquid insult without leaking, but, especially if the liquid is menses, the liquid will be easily visible throughout the article. This is not preferred by consumers and, as previously mentioned, may cause irritation of the skin if the liquid stays in contact with the skin. However, even if no irritation occurs, the look of the article after the insult is not aesthetically pleasing.

A number of U.S. patents address different problems associated with absorbent composites. For example, U.S. Patent No. 5,147,343 issued to Kellenberger teaches the importance of having a superabsorbent with high Absorbency Under Load values in an absorbent product. U.S. Patent No. 5,149,335 issued to Kellenberger et al. teaches the importance of superabsorbent rate and capacity in a composite. U.S. Patent No. 5,415,643 issued to Melius et al. teaches the importance of AUL values under different pressures. U.S. Patent No. 5,599,335 issued to Goldman emphasizes the benefits of the combination of high Saline Flow Conductivity and high Performance Under Pressure. U.S. Patent No. 5,728,082 issued to Gustafsson et al describes an absorbent body consisting of two layers containing superabsorbent, wherein the superabsorbent in the first layer has a high degree of cross-linking while the superabsorbent in the second layer has a higher absorbent capacity than the superabsorbent in the first layer.

The aforementioned patents disclose specific superabsorbent properties, which result in improved composite performance. In general, the aforementioned patents teach that superabsorbent materials exhibiting high capacity under load result in improved gel stiffness and permeability behavior for enhanced composite performance. However, the aforementioned patents still have not been able to produce an absorbent article, such as a diaper or feminine pad, that provide maximum protection, do not leak, stay dry to the touch, and, in the case of feminine pads, provide better fit with the wearer.

US 5,364,382 discloses an absorbent article including a retention portion for storing absorbed liquids and a surge management portion.

US 5,785,697 discloses a fluid handling member where lower portions are more dense than upper portions.

Accordingly, there is a need for an absorbent article that includes an improved absorbent structure that provides maximum protection to the wearer of the article. There is also a need for an absorbent article that includes an improved absorbent structure that handles liquids effectively while maintaining a relatively small size whether or not the absorbent structure incorporates superabsorbent materials. A need exists for an absorbent article that has an improved absorbent structure that stays dry to the touch. Additionally, there is a need for absorbent article that has an improved absorbent structure that provides a better fit with the wearer of the article. Finally, what is needed is an absorbent article that is more visually aesthetically pleasing, even after the absorbent article has been subjected to an insult.

The above-identified needs are addressed by the present invention that is directed to a multi-component liquid absorbent structure. The structure includes:
a first liquid absorbent component in the form of a coherent, flexible matrix including stratified layers of fibrous material, the first liquid absorbent component having a first major surface, a second major surface, a first total area and a first liquid absorbent capacity, and
a second liquid absorbent component containing at least one layer of a different liquid absorbent material adjacent the second major surface of the first liquid absorbent component, the second liquid absorbent component having a second total area and a second liquid absorbent capacity,
such that the ratio of the second total area to the first total area is greater than about 3.5 to 1, the ratio of the second liquid absorbent capacity to the first liquid absorbent capacity is greater than about 10 to 1 and an overall liquid absorbent capacity of the liquid absorbent structure is greater than about 35 grams.

According to the present invention, the first liquid absorbent component provides a distribution of interstitial spaces that are smaller in size adjacent the second major surface and larger in size adjacent the first major surface. For example, the interstitial spaces adjacent the first major surface may be about 1.25 times larger than the interstitial spaces adjacent the second major surface. This difference in size may range from about 1.25 to 1 up to about 3 to 1. Desirably, this difference in size may range from about 1.5 to 1 up to about 2.5 to 1.

In accordance with the invention, the first liquid absorbent component provides a distribution of interstitial spaces that are smaller in size adjacent the second major surface than interstitial spaces in the second liquid absorbent component adjacent that second major surface.

Generally speaking, the liquid absorbent capacity of the first liquid absorbent component may range from about 3 grams up to about 10 grams. A liquid absorbent capacity for the first liquid absorbent component of about 6 grams is desirable. The liquid absorbent capacity of the second liquid absorbent component may range from about 35 grams up to about 600 grams. For example, the liquid absorbent capacity of the second liquid absorbent component may range from about 35 grams up to about 150 grams. A liquid absorbent capacity for the second liquid absorbent component of about 100 grams is desirable.

Preferably, the first liquid absorbent component has a first volume, and the second liquid absorbent component has a second volume such that the ratio of the second volume to the first volume is greater than about 10 to 1 and the total volume of the liquid absorbent structure is less than about 130 cm³.

The ratio of the second area to the first area may range from about 3.5 to 1 up to about 8 to 1. Desirably , this ratio is about 4 to 1.

As another example, the ratio of the second volume to the first volume may range from about 10 to 1 up to about 100 to 1. In embodiments of the invention where the second liquid absorbent component utilizes relatively high levels of superabsorbent and is relatively thin, the ratio of the second volume to the first volume may range from about 10 to 1 up to about 30 to 1 and may desirably be about 16 to 1. In embodiments of the invention where the second liquid absorbent component utilizes relatively high levels of fluff pulp, the ratio of the second volume to the first volume may range from about 30 to 1 up to about 100 to 1. As yet another example, the ratio may desirably range from about 50 to 1 up to about 75 to 1. As an even further example, the ratio may desirably be about 75 to 1.

In an aspect of the invention, the total volume of the liquid absorbent structure is less than about 130 cm³. Desirably, the total volume of the liquid absorbent structure is less than about 100 cm³. More desirably, the total volume of the liquid absorbent structure is less than about 50 cm³. Still more desirably, the total volume of the liquid absorbent structure is less than about 30 cm³. Even more desirably, the total volume of the liquid absorbent structure is less than about 25 cm³.

According to the invention, the first liquid absorbent component may have strata composed of air-laid staple length fibers, air-laid fluff cellulose fibers, air-laid chemically modified cellulose fibers, hydrogel fibers and combinations thereof. In an aspect of the invention the generally stratified layers of the first liquid absorbent component comprises at least two layers of a fibrous nonwoven web. Desirably, at least one of the layers of a fibrous nonwoven web is selected from bonded-carded webs, air-laid webs, meltblown fiber webs, spunbonded filament webs, hydraulically entangled fiber webs and combinations thereof.

In an aspect of the invention, the first liquid absorbent component further includes particulate materials. These particulate materials may be superabsorbent materials (also referred to as such as hydrogel materials)

In order for the first liquid absorbent component to be a coherent matrix of fibrous material, it may be bonded utilizing thermal binder fibers, adhesives, thermal point bonding, mechanical entanglement, latex emulsions and combinations thereof.

The second liquid absorbent component may be selected from hydrogel containing composite structures, cellulose fluff structures, and generally homogeneous air-laid structures.

In one aspect of the invention, it is desirable that the interstitial spaces at the second major surface of the first liquid absorbent component are configured to transfer liquid substantially along the length of the first liquid absorbent component in addition to releasing liquid to the second liquid absorbent component.

In another aspect of the invention, the first liquid absorbent component retains less than about 30% of an artificial menses liquid about 1 minute after introduction of an approximately 10 mL insult at the center of the first major surface of the first liquid absorbent component. For example, the first liquid absorbent component retains less than about 25% of an artificial menses liquid about 1 minute after an approximately 10 mL insult. Desirably, the first liquid absorbent component retains less than about 20% of an artificial menses liquid about 1 minute after an approximately 10 mL insult. Even more desirably, the first liquid absorbent component retains less than about 15% of an artificial menses liquid about 1 minute after an approximately 10 mL insult.

In another aspect of the present invention, the first liquid absorbent component overlays or covers at least about 50% of a stain area in the second liquid absorbent component generated by artificial menses liquid about 10 minutes after an approximately 5 mL insult was introduced at the center of the first major surface of the first liquid absorbent component. For example, the first liquid absorbent component overlays or covers at least about 70% of a stain area in the second liquid absorbent component generated by artificial menses liquid about 10 minutes after an approximately 5 mL insult was introduced at the center of the first major surface of the first liquid absorbent component. As another example, first liquid absorbent component overlays or covers from about 75% to about 100% of a stain area in the second liquid absorbent component generated by artificial menses liquid about 10 minutes after an approximately 5 mL insult was introduced at the center of the first major surface of the first liquid absorbent component. As yet another example, the first liquid absorbent component overlays or covers from about 80% to about 100% of a stain area in the second liquid absorbent component generated by artificial menses liquid about 10 minutes after an approximately 5 mL insult was introduced at the center of the first major surface of the first liquid absorbent component.

The ratio of the second liquid absorbent capacity to the first liquid absorbent capacity may range from about 3.5 to 1 up to about 200 to 1. Desirably, this ratio will be in the range from about 10 to 1 up to about 30 to 1. More desirably, this ratio will be about 16 to 1.

Preferred embodiments of the present invention also encompass an absorbent personal care product incorporating the above-described absorbent structure. The absorbent personal care product may be in the form of a sanitary napkin. For example, in one embodiment, a sanitary napkin may have a longitudinal centerline and improved liquid management based on the utility of an absorbent multi-component structure, the sanitary napkin may include:
a liquid permeable body-facing layer;
a liquid impermeable garment-facing layer; and
a multi-component absorbent structure between the liquid permeable body-facing layer and the liquid impermeable garment-facing layer, the multi-component absorbent structure having:
   a first liquid absorbent component in which at least a portion of the component is positioned on the longitudinal centerline of the sanitary napkin, the first liquid absorbent component in the form of a coherent, flexible matrix of fibrous materials forming generally stratified layers, the first liquid absorbent component having a first major surface adjacent the liquid permeable body facing layer, a second major surface opposite the first major surface, a first total area and a first liquid absorbent capacity, and
   a second liquid absorbent component including at least one layer of a different liquid absorbent material adjacent the second major surface of the first liquid absorbent component, the second liquid absorbent component having a second total area and a second liquid absorbent capacity such that the ratio of the second total area to the first total area us greater than about 3.5 to 1 and the ratio of the second liquid absorbent capacity to the first liquid absorbent capacity is greater than about 10 to 1 and a total liquid absorbent capacity of the multi-component liquid absorbent structure is greater than about 35 grams.

The first liquid absorbent component and the second liquid absorbent component of the multi-component liquid absorbent structure in the sanitary napkin and any related properties and/or ratios may be in the form as generally described above.

The sanitary napkin may further include a channel in the second liquid absorbent component spanning at least a portion of the periphery of the first liquid absorbent component. Desirably, the channel surrounds the entire periphery of the first liquid absorbent component. The channel, which may be approximately oval or elliptical in shape, creates a configuration wherein the area inside the channel is at a raised level in relation to the rest of the sanitary napkin. As the raised area will be the part of the sanitary napkin closest to the wearer, the channel helps provide a better fit. Additionally, the channel prevents liquid from leaking out of the sanitary napkin.

In yet another preferred embodiment, the present invention encompasses a thin, efficient liquid absorbent structure. The structure includes:
a first liquid absorbent component including a coherent, flexible matrix including stratified layers of fibrous materials, the first liquid absorbent component having a first major surface, a second major surface, a first total area, thickness and volume and a first liquid absorbent capacity, and
a second liquid absorbent component including a layer of a different liquid absorbent material adjacent the second major surface of the first liquid absorbent component, the second liquid absorbent component having a second total area, thickness and volume and a second liquid absorbent capacity,
so that the thickness of first and second liquid absorbent component is each between about 1 mm and about 2 mm, the ratio of the second liquid absorbent capacity to the first liquid absorbent capacity is greater than about 10; the ratio of the second total area to the first total area is greater than about 3.5 to 1, the ratio of the second volume to the first volume is greater than about 10 to 1 and the total volume of the liquid absorbent structure is less than about 30 cm³, such that the first liquid absorbent component retains less than about 30% of an artificial menses liquid about 1 minute after an approximately 10 mL insult. For example, the first liquid absorbent component retains less than about 25% of an artificial menses liquid about 1 minute after an approximately 10 mL insult. Desirably, the first liquid absorbent component retains less than about 20% of an artificial menses liquid about 1 minute after an approximately 10 mL insult.

The first liquid absorbent component and the second liquid absorbent component of the multi-component liquid absorbent structure of the thin, efficient liquid absorbent structure and any related properties and/or ratios may be in the form as generally described above.

These and other features and advantages of the present invention will become apparent after a review of the following drawings and detailed

### description of the invention.

### BRIEF DESCRIPTION OF THE DRAWINGS

Figures 1A, 1B, and 1C show a partial partition view, a cross-sectional view, and a top view, respectively, for an exemplary feminine pad according to a first embodiment of the present invention.
Figures 2A, 2B, and 2C show a partial partition view, a cross-sectional view, and a top view, respectively, for an exemplary feminine pad according to a second embodiment of the present invention.
Figures 3A, 3B, and 3C show a partial partition view, a cross-sectional view, and a top view, respectively, for an exemplary feminine pad according to a third embodiment of the present invention.
Figures 4A, 4B, and 4C show a partial partition view, a cross-sectional view, and a top view, respectively, for an exemplary feminine pad according to a fourth embodiment of the present invention.
Figures 5A, 5B, and 5C show a partial partition view, a cross-sectional view, and a top view, respectively, for an exemplary feminine pad according to a fifth embodiment of the present invention.
Figures 6A and 6B show how an exemplary feminine pad according to one embodiment of the present invention pulls an insult into the interior of the article and away from the surface of the article closest to the wearer of the article.
Figures 7A and 7B show how an exemplary feminine pad according to another embodiment of the present invention pulls an insult into the interior of the article and away from the surface of the article closest to the wearer of the article.
Figure 8 is a top view of a test apparatus for measuring the rate which an absorbent structure absorbs a liquid.
Figure 9 is a cross-sectional view of a test apparatus for measuring the rate which an absorbent structure absorbs a liquid.

### DETAILED DESCRIPTION

As used herein, the terms "nonwovens" and "nonwoven web" means a web having a structure of individual fibers or threads that are interlaid, but not in any identifiable, repeating pattern. Nonwoven webs have been, in the past, formed by a variety of processes such as, for example, meltblowing processes, spunbonding processes, and bonded carded web processes.

As used herein, the terms "particle," "particles," "particulate," "particulates" and the like generally refer to materials that are in the form of finely divided, discrete units such as, for example, granules, pulverulents, powders or spheres. Desired particle shapes include, for example, cubic, rod-like, polyhedral, spherical or semi-spherical, rounded or semi-rounded, angular, irregular, etc. Shapes having a large greatest dimension/smallest dimension ratio, like, needles, fibers and flakes, are also contemplated for use herein. The desired shaped particles may be coated (gel-coated, protein coated and the like having a particulate core, a porous solid core, a solid core, a semi-solid core, a liquid core, a semi-liquid core, a gaseous core, a semi-gaseous core or combinations thereof) or uncoated (porous solid, solid, semi-solid and the like). It should be noted that more than one kind of particle may be used in some webs of the invention, either in mixture or in different layers. The use of "particle" and "particulate" may also describe an agglomeration comprising more than one particle, particulate or the like.

As used herein, the terms "superabsorbent" and "hydrogel" generally refer to absorbent materials capable of absorbing at least about 10 grams of aqueous liquid (e.g., water, saline solution or synthetic urine Item No. K-C 399105 available from PPG Industries) per gram of the absorbent material while immersed in the liquid for 4 hours and holding the absorbed liquid while under a compression force of up to about 0.5 pounds per square inch.

As used herein, the term "spunbond web" refers to a web formed by extruding a molten thermoplastic material as filaments from a plurality of fine, usually circular, capillaries with the diameter of the extruded filaments then being rapidly reduced, for example, by fluid-drawing or other well known spunbonding mechanisms. The production of spunbond nonwoven webs is illustrated in patents such as Appel, et al., U.S. Patent No. 4,340,563.

As used herein, the term "meltblown web" means a web having fibers formed by extruding a molten thermoplastic material through a plurality of fine, usually circular, die capillaries as molten fibers into a high-velocity gas (e.g. air) stream which attenuates the fibers of molten thermoplastic material to reduce their diameters. Thereafter, the meltblown fibers are carried by the high-velocity gas stream and are deposited on a collecting surface to form a web of randomly disbursed fibers. The meltblown process is well-known and is described in various patents and publications, including NRL Report 4364, "Manufacture of Super-Fine Organic Fibers" by V.A. Wendt, E.L. Boone, and C.D. Fluharty; NRL Report 5265, "An Improved Device for the Formation of Super-Fine Thermoplastic Fibers" by K.D. Lawrence, R.T. Lukas, and J.A. Young; and U.S. Patent No. 3,849,241, issued November 19, 1974, to Buntin, et al., which are hereby incorporated by reference.

As used herein, the term "fiber" refers to a fundamental solid form, usually semi-crystalline, characterized by relatively high tenacity and an extremely high ratio of length to diameter, such as several hundred to one. Exemplary natural fibers are wool, silk, cotton, and hemp. Exemplary semisynthetic fibers include rayon. Exemplary synthetic fibers include spinneret extruded polyamides, polyesters, acrylics, and polyolefins.

As used herein, the terms "airlaying", "air-laid", "air-formed", "air-forming" and the like refer to generally well-known processes by which a fibrous nonwoven layer can be formed by entraining small fibers in an air stream. In exemplary processes, bundles of small fibers having typical lengths ranging from about 3 to about 19 millimeters (mm) are separated and entrained in an air supply and then deposited onto a forming screen, usually with the assistance of a vacuum supply. The randomly deposited fibers may be then bonded to one another using, for example, hot air or a spray adhesive, Airlaying techniques are taught in, for example, US Patent 4,640,810 to Laursen et al.

The present invention provides a multi-component absorbent structure that incorporates a first liquid absorbent component generally in the form of a coherent flexible matrix including stratified layers of fibrous materials at or near the surface of the multi-component structure that is likely to be insulted or exposed to liquid. Such a construction provides several advantages. The first liquid absorbent component acts to draw an insult through the surface of the structure such that the insult is pulled into the underlying absorbent material where it is absorbed. As such, the first liquid absorbent component aids in the speed at which an insult is absorbed. Secondly, the first liquid absorbent component helps prevent any backflow of the insult after it has been pulled through. In this way, the multi-component liquid absorbent structure helps stabilize liquid in the structure and reduce the likelihood of leakage.

Additionally, since the insult is not able to travel back through the surface of the structure, the structure remains dry to the touch and a major portion of the insult is unable to migrate to surfaces that may be in contact with the first liquid absorbent component. For example, the absorbent structure reduces the flowback or rewet of the top surface of the absorbent structure that may come into contact with the skin of the wearer, thereby preventing irritation of the wearer's skin.

Also, the general configuration of the multi-component absorbent structure and the relative ratios of the volume and/or surface areas of the components help provide better performance and a better fit of the article with the wearer. For example, it has been found that the ratio of the area of the second liquid absorbent component to the area of the first liquid absorbent component is desirably greater than about 3.5 to 1 and that the ratio of the volume of the second liquid absorbent component to the volume of the first liquid absorbent component is desirably greater than about 10 to 1 - especially when the total volume of the liquid absorbent structure is less than about 130 cm³. Desirably, the total volume of the liquid absorbent structure is less than about 100 cm³. More desirably, the total volume of the liquid absorbent structure is less than about 50 cm³. Still more desirably, the total volume of the liquid absorbent structure is less than about 30 cm³. Even more desirably, the total volume of the liquid absorbent structure is less than about 25 cm³.

Finally, since a major portion of the insult is pulled into the second liquid absorbent component and does not remain near the surface of the first liquid absorbent component, the insult is in effect "hidden" from view except for a small spot on the surface of the article. As such, the article is more aesthetically pleasing after an insult.

For example, it has been found that when the components have been arranged in the ratios described above, the first liquid absorbent component retains less than about 30% of an artificial menses liquid about 1 minute after an approximately 10 mL insult. Desirably, the level of retention of artificial menses liquid is less than about 25%, less than about 20%, or even less than about 15% about 1 minute after an approximately 10 mL insult.

With respect to stain coverage or masking, it has also been found that when the components have been arranged in the ratios described above, the first liquid absorbent component desirably overlays or covers at least about 50% of a stain area in the second liquid absorbent component generated by artificial menses liquid about 10 minutes after an approximately 5 mL insult introduced at the center of the first major surface of the first liquid absorbent component. The percentage of stain overlay or stain coverage may be at least about 70% and may desirably range from about 75% to about 100% of a stain area in the second liquid absorbent component generated by artificial menses liquid about 10 minutes after an approximately 5 mL insult introduced at the center of the first major surface of the first liquid absorbent component.

The present invention provides an absorbent article such as, for example, a sanitary napkin that provides maximum protection, does not leak and stays dry to the touch, provides a better fit, and, after an insult, are more aesthetically pleasing than prior art absorbent articles. The absorbent articles contain an improved absorbent structure that has been developed to achieve these improvements. Additionally, the present invention includes a channel located around an interior portion of the absorbent article. The channel helps provide a center-raised device that provides a better fit with the wearer of the article.

The absorbent structure of the present invention includes a first liquid absorbent component that is a coherent, flexible matrix including stratified layers of fibrous material. This first liquid absorbent component serves several purposes. Initially, the first major surface of the first liquid absorbent component acts like a "surge" or rapid intake layer to rapidly absorb liquid. Then, subsequent strata in the first liquid absorbent component serve to wick or transport liquid from adjacent the surface to the interior and/or lowest strata of the first liquid absorbent component. During operation, liquid is transferred from the first liquid absorbent component to second liquid absorbent component or "absorbent core" where it is stored in the underlying absorbent material. Additionally, since liquid has been rapidly pulled into the interior of the multi-component liquid absorbent structure, a top view of the article should generally reveal a stain pattern that shows only as a very small circle, thereby giving the wearer the perception of the article being clean. Finally, since the liquid is stored in the second liquid absorbent component in the interior of the structure, the surface of the article is generally drier to the touch and less of the liquid is able to contact the surface of the structure, helping to prevent irritation of the skin of the wearer.

The multi-component liquid absorbent structure used in the present invention includes a first liquid absorbent component that is a coherent, flexible matrix including stratified layers of fibrous material and a second liquid absorbent component that may be a more traditional absorbent material such as a batt of cellulose fluff, superabsorbent material containing sheet or combinations thereof.

The coherent, flexible matrix including stratified layers of fibrous material desirably has a relatively open layer at the top-most or first major surface. The open layer acts as the surge layer. Additionally, the open layer on top helps to prevent the flow back of liquid once it has been trapped and absorbed by the structure, thereby providing a dry feel, helping to prevent leakage, and helping reduce the potential for irritation should the liquid flow back and contact the wearer of the article.

Additionally, the coherent, flexible matrix including stratified layers of fibrous material desirably includes a wicking layer to help pull liquid from the open layer through the strata and into the interior of the first liquid absorbent component and ultimately into the absorbent article so that a major portion of the liquid gets absorbed and stored by the second liquid absorbent component.

While the inventors should not be held to a particular theory of operation, it is thought that the first liquid absorbent component provides a distribution of interstitial spaces that are smaller in size adjacent the second major surface and larger in size adjacent the first major surface. This difference in the size of interstitial spaces is believed to provide a graduated or gradient distribution that helps to draw liquid from the first major surface to the second major surface. As an example, the interstitial spaces adjacent the first major surface may be about 1.25 times larger than the interstitial spaces adjacent the second major surface. This difference in size may range from about 1.25 to 1 up to about 3 to 1.

According to the invention, the first liquid absorbent component provides a distribution of interstitial spaces that are smaller in size adjacent the second major surface than interstitial spaces in the second liquid absorbent component adjacent that second major surface. Such a configuration is thought to slow the transfer of liquid from the first liquid absorbent component to the second liquid absorbent component. While the inventors should not be held to a particular theory of operation, it is thought that the difference in size of interstitial spaces between the two components causes more liquid to spread throughout the second major surface of the first liquid component before transfer to second liquid absorbent component. Such operation is thought to be advantageous when the second liquid absorbent component includes superabsorbent material that has a relatively low rate of intake.

When the liquid absorbent structure is incorporated into an absorbent article such as, for example, a sanitary napkin, at least a portion of the first liquid absorbent component is desirably located in a central region (e.g., along a longitudinal centerline) of the article closest to the wearer. In this position, the first liquid absorbent component acts to cause a raised center of the article, therefore providing a better fit of the article with the wearer. This better fit may be enhanced through the use of a channel located around an interior portion of the article (the interior portion including the first liquid absorbent component). The channel reduces the bulk of the article at areas where the article is mostly not being used, further enhancing the raised center of the article and, therefore, further providing a better fit of the article with the wearer. The channel also helps to prevent leaking of any liquid absorbed in the absorbent article.

Reference is now made to the Figures of the drawings that illustrate several alternative embodiments for exemplary absorbent articles of the present invention. These particular exemplary embodiments are sanitary napkins or feminine pads that incorporate the above-described liquid absorbent structure. Of course, it should be understood that a variety of absorbent articles may successfully employ the liquid absorbent structure.

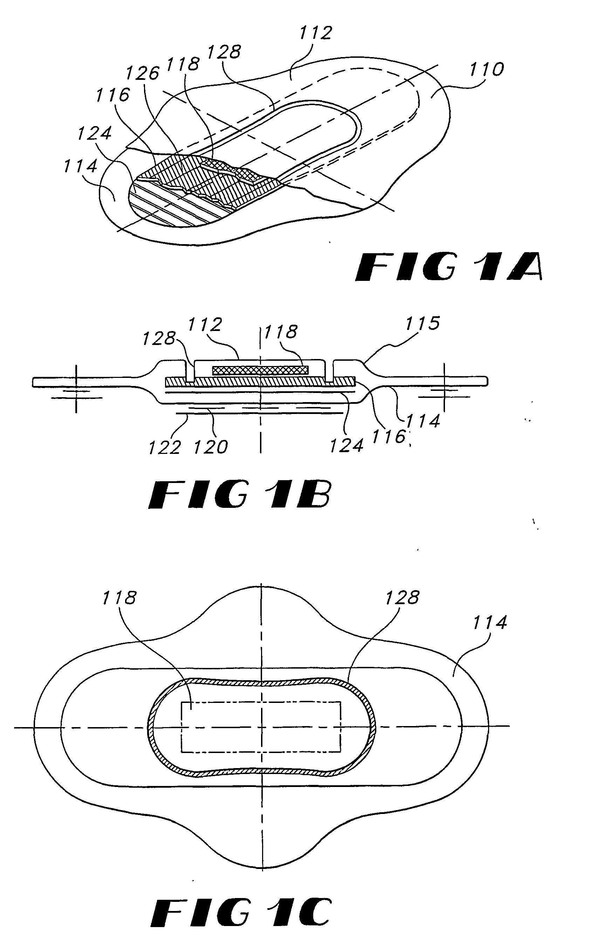

Referring now to Figures **1a****-c,** there is shown an exemplary feminine pad or sanitary napkin **110** that is a large capacity maxi-style pad that includes wings. As shown in Figures **1a** and **1b****,** the feminine pad **110** includes a cover or topsheet **112** that may be a film, nonwoven material or combinations thereof. The pad also includes a breathable polymer or plastic film baffle or backsheet **114** that may be configured to have flaps or wings extending outward from the central section of the pad. The pad further includes a liquid absorbent structure **115** which includes a second liquid absorbent component **116** and a first liquid absorbent component **118** that is a coherent, flexible matrix including stratified layers of fibrous material. The first liquid absorbent component **118** is located under the film cover **112** and above the second liquid absorbent component **116.** As previously discussed, the first liquid absorbent component **118** acts to pull liquid through the cover **112,** past the first liquid absorbent component **118** and into the second liquid absorbent component **116,** where it is absorbed and stored.

The feminine pad **110** may also include other features such as garment adhesive material **120** to hold the pad in place, a release liner **122** for covering the adhesive **120,** a tissue layer **124** beneath the liquid absorbent structure **115** to aid in absorbency, and even embossing **126** in the second liquid absorbent component **116** to selectively draw and store liquid in a desired location. In some embodiments, it is contemplated that the tissue layer **124** may be located between the first liquid absorbent component **118** and the second liquid absorbent component **116.**

The feminine pad **110** may also include a channel **128** that is located on an interior portion of the feminine pad **110,** yet surrounds the first liquid absorbent component **118.** As discussed, the channel **128** helps create a feminine pad **110** that fits better than conventional feminine pads. The channel may be in the second liquid absorbent component and may span at least a portion of the periphery of the first liquid absorbent component.

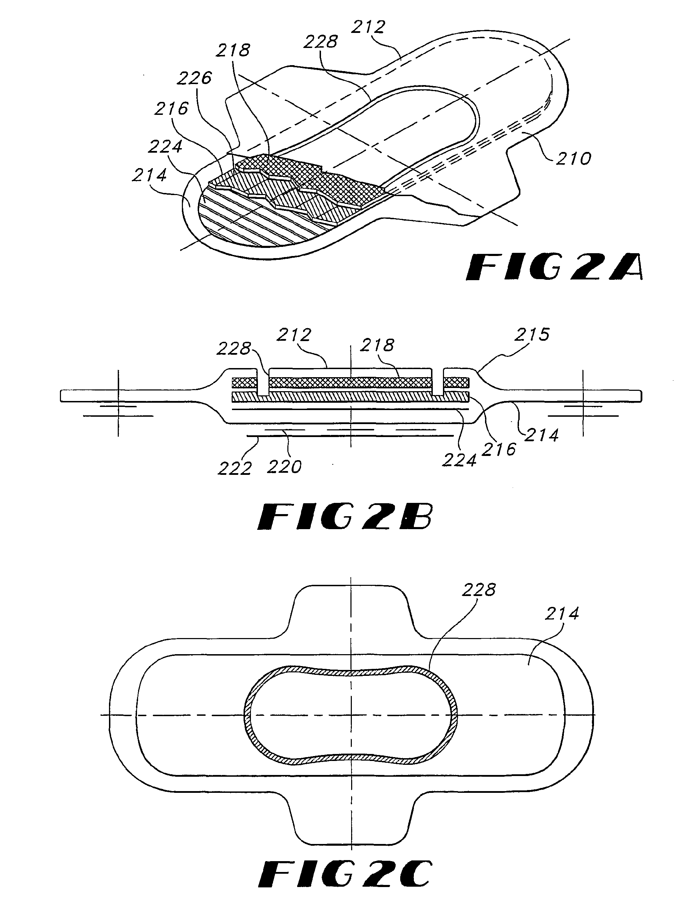

Figures **2a****-c** show an exemplary feminine pad or sanitary napkin **210** that illustrates a "slim" style construction and also includes wings. The feminine pad **210** includes a cover **212** that may be a film, nonwoven material or combinations thereof. The pad also includes a breathable polymer or plastic film baffle or backsheet **214** that may be configured to have flaps or wings extending outward from the central section of the pad.

The pad further includes a liquid absorbent structure **215** which includes a second liquid absorbent component **216** and a first liquid absorbent component **218** that is a coherent, flexible matrix including stratified layers of fibrous material. The first liquid absorbent component **218** is located under the cover **212** and above the second liquid absorbent component **216.** As previously discussed, the first liquid absorbent component **218** acts to pull liquid through the cover **212,** past the first liquid absorbent component **218** and into the second liquid absorbent component **216,** where it is absorbed and stored.

The feminine pad **210** may also include other features such as garment adhesive material **220** to hold the pad in place, a release liner **222** for covering the adhesive **220,** a tissue layer **224** beneath the liquid absorbent structure **215** to aid in absorbency, and even embossing **226** in the second liquid absorbent component **216** to selectively draw and store liquid in a desired location. In some embodiments, it is contemplated that the tissue layer **224** may be located between the first liquid absorbent component **218** and the second liquid absorbent component **216.**

The feminine pad **210** may also include a channel **228** that is located on an interior portion of the feminine pad **210,** yet surrounds the first liquid absorbent component **218.** As discussed, the channel **228** helps create a feminine pad **210** that fits better than conventional feminine pads. The channel may be in the second liquid absorbent component and may span at least a portion of the periphery of the first liquid absorbent component.

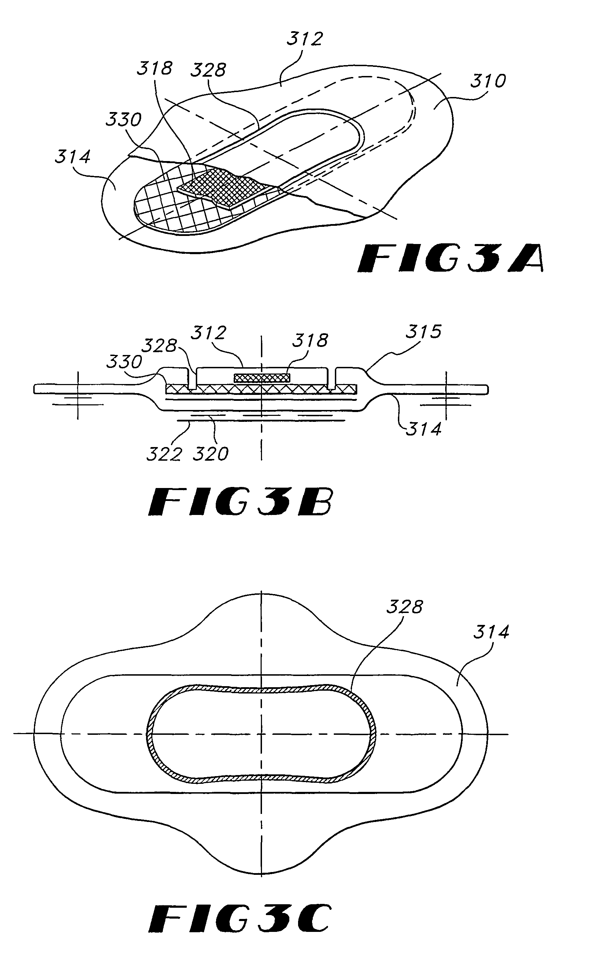

Figures **3a****-c** show an exemplary feminine pad or sanitary napkin **310** that illustrates an "ultra-slim" or "ultra-thin" style construction and also includes wings. The feminine pad **310** includes a cover **312** that may be a film, nonwoven material or combinations thereof. The pad also includes a breathable polymer or plastic film baffle or backsheet **314** that may be configured to have flaps or wings extending outward from the central section of the pad.

The pad further includes a liquid absorbent structure **315** which includes a second liquid absorbent component **330** and a first liquid absorbent component **318** that is a coherent, flexible matrix including stratified layers of fibrous material. The first liquid absorbent component **318** is located under the cover **312** and above the second liquid absorbent component **330.**

In this embodiment, the second liquid absorbent component **330** may be a thin sheet of superabsorbent containing material. The second liquid absorbent component **330** may be wrapped or surrounded or sandwiched by a tissue layer. It is contemplated that he second liquid absorbent component **330** in this "ultra-slim" or "ultra-thin" construction may be replaced by a large piece of the material used as the first liquid absorbent component **318.**

The feminine pad **310** set forth in Figures **3a****-c** may also include an adhesive material **320** and a release liner **322.** Finally, the feminine pad **310** may also include a channel **328** located on an interior portion of the feminine pad **310.** The channel may be in the second liquid absorbent component and may span at least a portion of the periphery of the first liquid absorbent component.

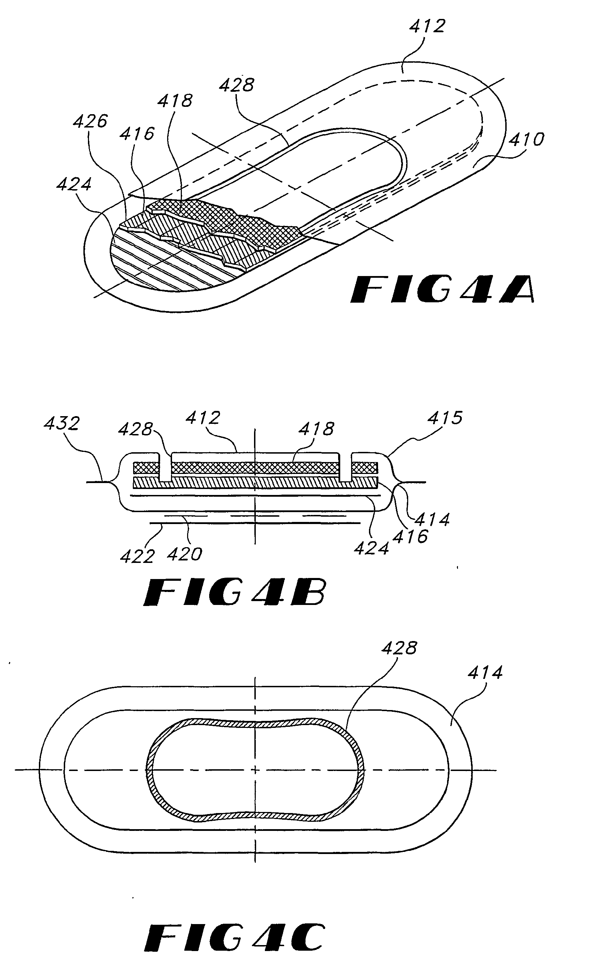

Figures **4a****-c** show an exemplary feminine pad or sanitary napkin **410** that illustrates a "maxi" style construction without wings. The feminine pad **410** includes a cover or topsheet **412** that may be a film, nonwoven material or combinations thereof. The pad also includes a breathable polymer or plastic film baffle or backsheet **414.** The pad further includes a liquid absorbent structure **415** which includes a second liquid absorbent component **416** and a first liquid absorbent component **418** that is a coherent, flexible matrix including stratified layers of fibrous material. The first liquid absorbent component **418** is located under the film cover **412** and above the second liquid absorbent component **416.**

The feminine pad **410** also includes an adhesive material **420,** a release liner **422** for covering the adhesive **420,** a tissue **424,** embossing **426,** and a seal **432** around the exterior of the pad **410.** Finally, the feminine pad **410** also includes a channel **428** located on an interior portion of the feminine pad **410.** As can bee seen in Figure **4b****,** however, the channel **428** does not completely enclose the area of the pad **410** containing the first liquid absorbent component **418.** The channel may be in the second liquid absorbent component and may span at least a portion of the periphery of the first liquid absorbent component.

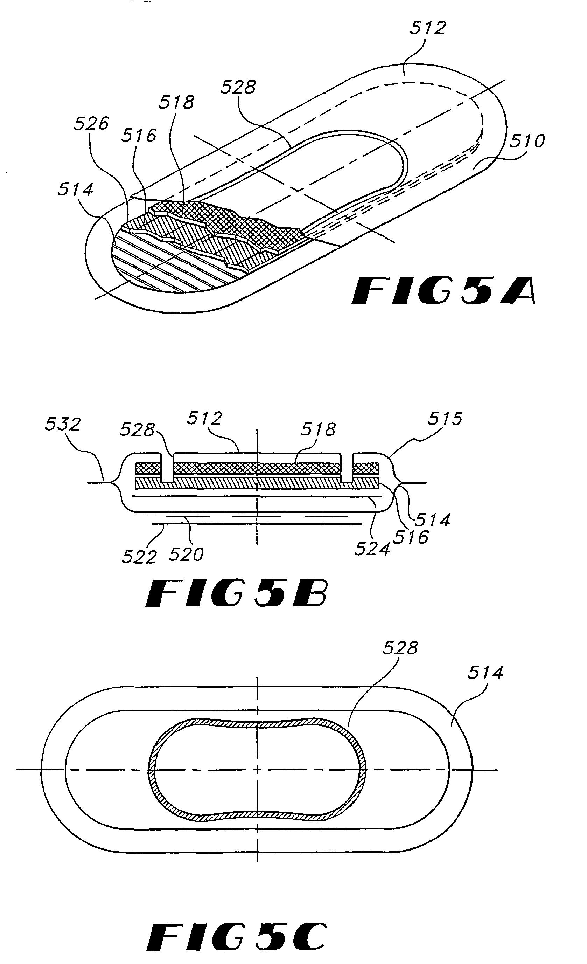

Figures **5a****-c** an exemplary feminine pad or sanitary napkin **510** that illustrates a "slim" style construction without wings. The feminine pad **510** includes a cover or topsheet **512** that may be a film, nonwoven material or combinations thereof. The pad also includes a breathable polymer or plastic film baffle or backsheet **514.** The pad further includes a liquid absorbent structure **515** which includes a second liquid absorbent component **516** and a first liquid absorbent component **518** that is a coherent, flexible matrix including stratified layers of fibrous material. The first liquid absorbent component **518** is located under the film cover **512** and above the second liquid absorbent component **516.**

The feminine pad **510** also includes an adhesive material **520,** a release liner **522** for covering the adhesive **520,** a tissue **524,** embossing **526,** and a seal **532** around the exterior of the pad **510.** Finally, the feminine pad **510** also includes a channel **528** located on an interior portion of the feminine pad **510.** As can bee seen in Figure **5b****,** however, the channel **528** does not completely enclose the area of the pad **510** containing the first liquid absorbent component **518.** The channel may be in the second liquid absorbent component and may span at least a portion of the periphery of the first liquid absorbent component.

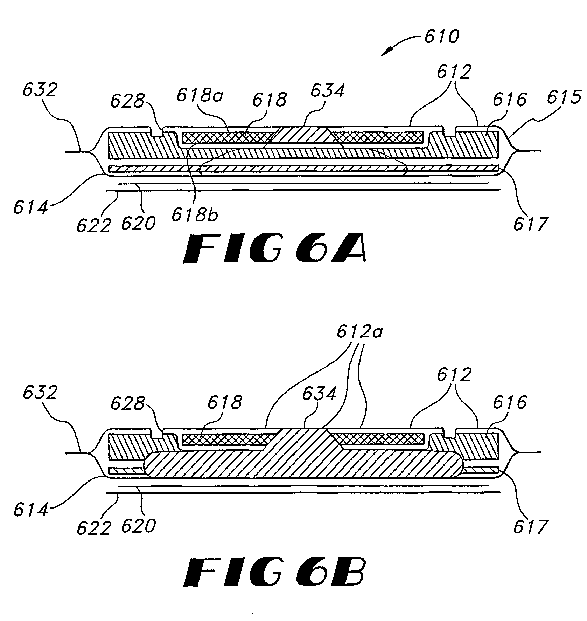

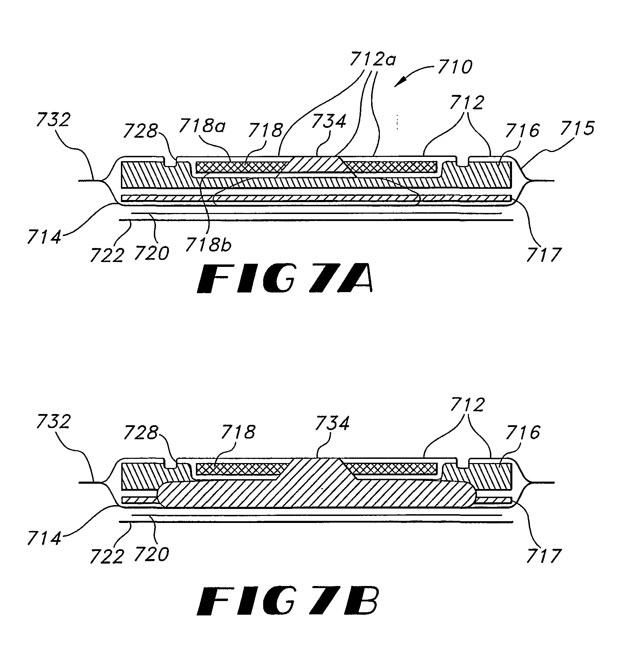

Turning now to Figures **6a****-b** and **7a-b,** the advantages of having a first liquid absorbent component that is a coherent, flexible matrix including stratified layers of fibrous material and a second liquid absorbent component in an absorbent article will be shown in connection with two different types of feminine pad.

Figure **6a** shows a feminine pad or sanitary napkin **610** having a nonwoven cover **612** and a plastic film baffle **614.** The pad has a liquid absorbent structure **615** that includes a second liquid absorbent component **616,** a first liquid absorbent component **618,** and an optional lower layer of an absorbent material such as, for example, a tissue sheet **617.** The first liquid absorbent component **618** is a coherent, flexible matrix including stratified layers of fibrous material having a first major surface **618a** and a second major surface **618b.** The pad **610** further includes an adhesive **620,** a release liner **622,** a channel **628** and a seal **632.**

As shown by the arrow, a liquid insult **634,** in this instance, menses, is delivered to the pad **610.** The menses **634** passes through the cover **612** and contacts the first major surface **618a** of the first liquid absorbent component **618.** The relatively open or porous strata adjacent the first major surface **618a** of the first liquid absorbent component **618** acts as a surge layer to permit the rapid intake of liquid. Interior strata of the first liquid absorbent component **618** acts to draw the menses **634** through the first liquid absorbent component **618** and into the second liquid absorbent component **616** and optional lower layer **617.**

As shown in Figure **6b****,** the menses **634,** is absorbed into the second liquid absorbent component **616** and the optional lower layer **617,** but generally does not flow back through the first liquid absorbent component **618.** If the pad were viewed from the top, only a small portion **612a** of the pad **610** would appear to have been subjected to the menses **634,** making the pad **610** more aesthetically pleasing. Additionally, since the menses **634** does not flow back through the first liquid absorbent component **618,** the cover **612** feels dry to the touch and minimal contact of the menses **634** with the skin of the wearer is achieved, thereby reducing the possibility of irritation of the skin.

Figures **7a****-b** are similar to Figures **6a****-b,** except that a film cover **712** is used instead of a nonwoven cover. The feminine pad or sanitary napkin **710** includes a plastic film baffle **714.** The pad has a liquid absorbent structure **715** that includes a second liquid absorbent component **716,** a first liquid absorbent component **718,** and an optional lower layer of an absorbent material such as, for example, a tissue sheet **717.** The first liquid absorbent component **718** is a coherent, flexible matrix including stratified layers of fibrous material having a first major surface **718a** and a second major surface **718b.** The pad **710** also includes an adhesive **720,** a release liner **722,** a channel **728** and a seal **732.**

Again, as menses **734** is delivered to the pad **710,** the menses **734** passes through the cover **712** and contacts the first liquid absorbent component **718** which then draws the menses **734** through the first liquid absorbent component **718** and into the second liquid absorbent component **716** and the optional lower layer **717.**

The first liquid absorbent component can have various strata selected based upon the desired characteristics of the absorbent article. As previously discussed, the first liquid absorbent component desirably has an open top strata or layer that acts as a surge layer and helps prevent the flow back of liquid once it has been trapped and absorbed by the article. Additionally, the first liquid absorbent component desirably includes a transfer or wicking layer.

Many different types of coherent, flexible matrices including stratified layers of fibrous material may be used as the first liquid absorbent component. Examples of suitable materials that are useful in the present invention include several multifunction air laid materials available from Buckeye Technologies, Inc. (Memphis, Tennessee). One such material comprises an air-formed multi-strata web comprised of polyester (PET) fibers, fluff cellulose fibers and chemically modified cellulose fibers that are formed onto a carrier tissue sheet, which is then bonded with a combination of a PET/polyethylene bi-component binder fiber and an ethylvinyl alcohol-based latex emulsion.

Binders can be used to help provide mechanical integrity and stabilization. Binders include fiber, liquid or other binder means which may thermally activated. Desirable binder fibers include those having a relative melting point such as polyolefin fibers. Fibers having a lower melting polymer, like conjugate and biconstituent fibers are desirable. Fibers having a lower melting polymer are generally referred to as "fusible fibers". By "lower melting polymers" what is meant are those having a glass transition temperature less than about 175 C. It should be noted that the texture of the absorbent web could be modified from soft to stiff through selection of the glass transition temperature of the polymer. Exemplary binder fibers include conjugate fibers of polyolefins, polyamides and polyesters. Exemplary binder fibers include sheath core conjugate fibers available from KoSa Inc. (Charlotte, North Carolina) under the designation T-255 (Merge 34821 A) and T-256 or Copolyester designation, though many suitable binder fibers are known to those skilled in the art, and are available by many manufacturers such as Chisso and Fibervisions LLC of Wilmington, DE. KoSa has developed a suitable co-polyester binder fiber as a sheath core application and is known by designation T254 (low melt CoPET). A suitable liquid binder is KYMENE® 557LX available from Hercules Co. of Wilmington, DE. Other suitable liquid binders include ethylene vinyl acetate emulsion polymers sold by National Starch and Chemical Company (Bridgewater, New Jersey) under the tradename DUR-O-SET® ELITE® series (including ELITE® 33 and ELITE® 22). Air Products Polymers and Chemicals sells other suitable binder fibers under the name AIRFLEX®.

Synthetic fibers include those made from polyamides, polyesters, rayon, acrylics, superabsorbents, TENCEL® regenerated cellulose and any other suitable synthetic fibers known to those skilled in the art. Synthetic fibers may also include kosmotropes for product degradation.

Many polyolefins are available for fiber production, for example polyethylenes such as Dow Chemical's ASPUN® 6811A liner low density polyethylene, 2553 LLDPE and 25355 and 12350 high density polyethylene are such suitable polymers. The polyethylenes have melt flow rates, respectively, of about 26, 40, 25 and 12. Fiber forming polypropylenes include Exxon Chemical Company's ESCORENE® PD 3445 polypropylene and Montell Chemical Co.'s PF304. Many other polyolefins are also available. Particularly preferred materials for this application include polyesters, which may range in size or denier from 3 to 25 denier, and having various cross-sections including round, pentalobal, helical crimped, etc. Such fibers have been developed by KoSa, Inc. with a durably wettable finish and are known by designation of fiber denier followed by polymer type and cross section. Examples would include 8 dpf, T-224 (High Void); 8 dpf, T-224 (trilobal); 15 dpf T-224 (round); 10 dpf T-224 (round); 6 dpf T-224 (round) and 3 dpf T-224 (round).

Natural fibers include wool, cotton, flax, hemp and wood pulp. Wood pulps include standard softwood fluffing grade such as CR-1654 (US Alliance Pulp Mills, Coosa, Alabama). Pulp may be modified in order to enhance the inherent characteristics of the fibers and their processability. Curl may be imparted to the fibers by methods including chemical treatment or mechanical twisting. Curl is typically imparted before crosslinking or stiffening. Pulps may be stiffened by the use of crosslinking agents such as formaldehyde or its derivatives, glutaraldehyde, epichlorohydrin, methylolated compounds such as urea or urea derivatives, dialdehydes, maleic anhydride, non-methylolated urea derivatives, citric acid or other polycarboxylic acids. Some of these agents are less preferable than others due to environmental and health concerns. Pulp may also be stiffened use of heat or caustic treatments such as mercerization. Examples of these types of fibers include NHB416 which is a chemically crosslinked southern softwood pulp fibers which enhances wet modulus, available from the Weyerhaeuser Corporation of Tacoma, WA. Other useful pulps are debonded pulp (NF405) and non-debonded pulp (NB416) also from Weyerhaeuser. HPZ3 from Buckeye Technologies, Inc of Memphis, TN, has a chemical treatment that sets in a curl and twist, in addition to imparting added dry and wet stiffness and resilience to the fiber. Another suitable pulp is Buckeye HPF2 pulp and still another is IP SUPERSOFT® from International Paper Corporation. Suitable rayon fibers are 1.5 denier Merge 18453 fibers from Tencel Incorporated of Axis, Alabama.

As a more detailed example, an exemplary material suitable for the first liquid absorbent component is available from Buckeye Technologies, Inc. under the trade designation Buckeye Unicore 8001. This air-formed or air-laid multi-strata material may have a total basis weight in the range from about 120 to about 300 (desirably between about 210 and 240) grams per square meter (gsm) and an overall density ranging from about 0.06 to about 0.10 grams per cubic centimeter (g/cm³). Exemplary multi-strata materials may have a top or uppermost layer of about 25 to about 45 gsm that contains latex bonded polyester fibers ranging from about 6 to about 15 denier per fiber (dpf) and which desirably make up about 20% of the total basis weight of the strata in the first liquid absorbent component. An immediately adjacent layer may be about 35 to about 70 gsm and may contain cellulose fiber joined with a binder fiber. The cellulose may be mercerized cellulose that is thermally bonded utilizing a bicomponent polyester/polyethylene binder fiber. This layer may desirably make up about 30% of the total basis weight of the strata. The multi-strata material may further include a layer that is about 35 to about 100 gsm and may include a compressible cellulose and binder fiber of the same or similar type as in the adjacent layer. This other layer may make up about 40% of the total basis weight of the strata. These layers may be formed on or supported by a carrier tissue that may range from about 10 to about 20 gsm and may desirably make up about 5 percent of the total basis weight of the strata.

Other examples of suitable coherent, flexible matrices including stratified layers of fibrous material that may be used as the first liquid absorbent component can be found in International Publication Number WO 00/74620. According to that publication, the terms "strata" and "stratum" refer to the layered regions which make up a unitary structure. The strata of the unitary structure is not an assembly or laminate of preformed layers forming a multilayered structure. Instead, the unitary structure is constructed by assembling the strata in a continuous, manner. Airlaid technology is described as the method for assembling the strata of the unitary structure.

Another example of a suitable coherent, flexible matrix including stratified layers of fibrous material that may be used as the first liquid absorbent component has two strata or layers. The first layer is an air-laid structure having a basis weight of about 50 gsm and containing 85%, by weight, polyester fibers - 15 denier per filament that is bonded together with about 15%, by weight, of a conventional latex binder suitable for personal care products. The second layer is an air-laid structure having a basis weight of about 150 gsm and containing about 90%, by weight, cellulose fluff and about 10%, by weight, bi-component binder fibers composed of a polyester core and a polyethylene or polyethylene-like sheath that softens or melts when heat is applied to dry the latex binder and thermally activate the binder fibers. This specific combination layers had an overall thickness of about 1.6 millimeters.

The present invention is further illustrated by the following examples, which are not to be construed in any way as imposing limitations upon the scope thereof. On the contrary, it is to be clearly understood that resort may be had to various other embodiments, modifications, and equivalents thereof which, after reading the description herein, may suggest themselves to those skilled in the art without departing from the scope of the appended claims.

### EXAMPLES

### Example 1

In Example 1, feminine pads were tested to determine the effectiveness of sanitary napkins employing the multi-component liquid absorbent structure in comparison to a more conventional commercial feminine pad. In this Example, sanitary napkins incorporating a multi-component liquid absorbent structure were identified as GEM II. The same multi-component liquid absorbent structures were used in both a maxi-style pad and a slim-style pad.

The both the GEM II "maxi" and "slim" pads had a multi-component liquid absorbent structure in which the first liquid absorbent component had an area of about 38 square centimeters and the second liquid absorbent component had an area of 131 square centimeters.

The first liquid absorbent component had a thickness of approximately 1.5 millimeters (0.15 cm) to yield a calculated volume of about 6 cm³. The GEM II "maxi" pads utilized a second liquid absorbent component formed of cellulose fluff. This component had a thickness of about 8 millimeters (0.8 cm) to yield a calculated volume of about 105 cm³. The GEM II "slim" pads utilized a second liquid absorbent component formed of cellulose fluff. This component had a thickness of about 6 millimeters (0.6 cm) to yield a calculated volume of about 79 cm³.

The first liquid absorbent component was a material available from Buckeye Technologies under the trade designation Unicore 8001. The material has basis weight of approximately 230 gsm having a liquid absorbent capacity of approximately 6 grams of liquid per gram of material. For the "maxi" style pads, the second liquid absorbent component was a batt of fluff pulp having a basis weight of approximately 662 gsm and a liquid absorbent capacity of approximately 10 grams of liquid per gram of material. For the "slim" style pads, the second liquid absorbent component was a batt of fluff pulp having a basis weight of approximately 397gsm and a liquid absorbent capacity of approximately 10 grams of liquid per gram of material.

The control commercial feminine pads were Laurier brand Soft Care pads in both a maxi-style and slim-style. These pads are available in Malaysia from Kao Corporation. Generally speaking, the pads have a single liquid absorbent component that can be described as a tissue wrapped cellulose fluff core. The liquid absorbent component has a basis weight of about 519 gsm for the "maxi" product and about 323 gsm for the "slim" product. This liquid absorbent component is located between an apertured film cover and a liquid impermeable backsheet.

Table 1 sets forth the physical measurements as well as the results of testing for each of the six pads. Each of the six pads was tested for its absorbency using the following testing procedure with 10 cm³ (∼10 ml) of liquid added to each maxi pad and 6 cm³ (∼6 ml) of liquid added to each slim pad. The tests were conducted utilizing an artificial menstrual liquid described in U.S. Patent No. 5,833,231, the contents of which are incorporated herein by reference. A more detailed description of the artificial menses follows.

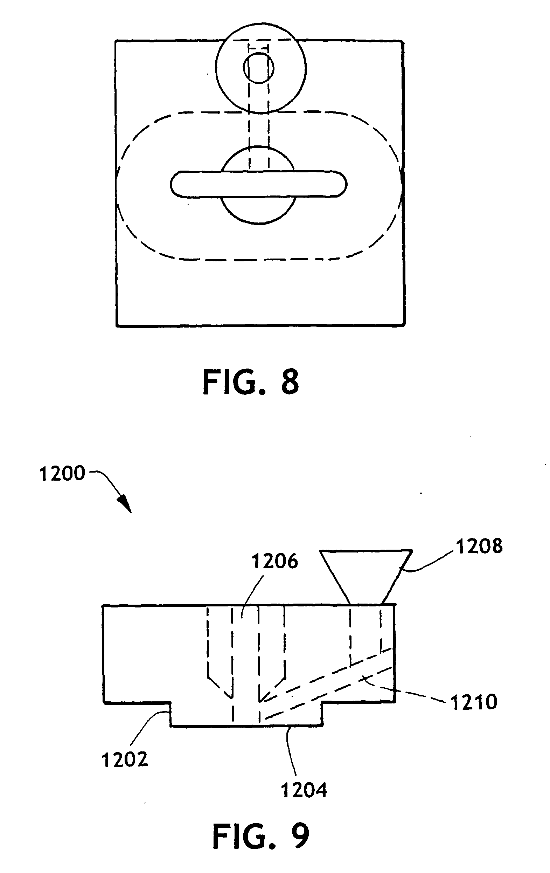

The tests utilized a test apparatus which consisted of: 1) a Lucite® block; and 2) a flat, horizontal test surface. Figure 8 is a plan view of the Lucite® block. Figure 9 is a sectional view of the Lucite® block. The block **1200** has a base **1202** that protrudes from the bottom of the block. The base **1202** has a flat surface **1204** which is approximately 2.875 inches long by 1.5 inches wide that forms the bottom of the block **1200.** An oblong opening **1206** (about 1.5 inches long by about 0.25 inch wide) is located in the center of the block and extends from the top of the block to the base **1202** of the block. When the bottom of the opening **1206** is obstructed, the opening **1206** can hold more than about 10 cm³ of liquid. A mark on the passage **1210** indicates a liquid volume of about 2 cm³. A funnel **1208** on the top of the block feeds into a passage **1210** that is connected to the oblong opening **1206.** Liquid poured down the funnel **1208** passes through the passage **1210** into the oblong opening **1206** and out onto a test sample underneath the block.

Each sample was tested by placing it on a flat, horizontal test surface and then putting the flat, projecting base of the block on top of the sample so that the long dimension of the oblong opening was parallel to the long dimension of the sample and centered between the ends and sides of the sample. The weight of the block was adjusted to about 162 grams so that so that the block rested on the structure with a pressure of about 7 grams/cm² (about 1 psi). A stopwatch was started as approximately ten (10) cm³ for maxi pads (or 6 cm³ for slim and ultrathin pads) of the liquid was dispensed into the funnel from a Repipet (catalog No. 13-687-20; Fischer Scientific Company) utilizing consistent pressure. The liquid filled the oblong opening of the block and the watch was stopped when the meniscus of the liquid reached the 2 cm³ volume level indicating that 8 cm³ of liquid was absorbed.

Additionally, a rewet test was performed utilizing a blotter rewet test to measure the amount of artificial menses, in grams, which exited the sample under a uniform application of 1 pound per square inch (psi) pressure. A pre-weighed piece of blotter paper, such as 100lb. Blotter - brand name "Verigood" available from Fort James (Georgia Pacific) having offices in Atlanta, GA; or 100# Blotter- brand name "Riegel" available from Sloan Paper Co. having offices in Roswell, Ga. or its equivalent, was placed on the sample. A uniform pressure of 1 psi then was applied to the pad for three minutes. The pressure was removed and the wet blotter paper was weighed to the nearest 0.01 gram. The amount of artificial menses desorbed from the sample onto the blotter paper was determined by subtracting the dry blotter paper weight from the wet blotter paper weight.

Absorbent capacity is determined by dispensing an aqueous dye solution into a specimen until the specimen leaks. The liquid amount dispensed at the point of leakage is the absorbent capacity. The equipment consists of a peristaltic tubing delivery pump system, (tubing pump, tubing, and a capillary cannula delivery needle), a control cabinet to simultaneously start and stop the pump and a timer, and a flat, horizontal probe sensing grid that will sense the presence of fluid. The pump delivers liquid until the specimen becomes totally saturated, and fluid drips from the specimen to the sensing grid. When this occurs, the control cabinet stops the pump and timer, and the amount of fluid is calculated by multiplying the fluid dispensed (mL) x time (min).

To prepare for the test, a concentrated solution is prepared by dissolving 114 mL of N0. 357 Blue Liquid Color (part No 00357) into 1000 mL +/- 5 mL of distilled water. The test solution is prepared by adding 30 +/- 1 mL of concentrate to 3400 +/- 5 mL of distilled water. The dye is supplied by Warner-Jenkinson Co., St. Louis, MO. The volume of fluid used for the testing is 1000 mL, which is stored in a 1000 mL graduated cylinder.

An automated, positive flow rate pump is used to pump the solution from the graduated cylinder to the specimen, e.g., a Masterflex (R) pump, part number 826028, with a Masterflex (R) pump head, part number 1034618, fitted with Masterflex(R) #14 silicone tubing, part number 96410-14, all available from Cole-Palmer Instrument Co., Chicago, IL. A four inch long hypodermic needle or laboratory cannula, e. g., 14 Ga. stainless steel cannula with luer-loc, part number BD1789, supplied by Becton Dickinson of Franklin Lakes, NJ. The 14 gauge needle has the same inner diameter as the tubing, and is added to make the set-up more rigid, which facilitates support of the delivery end of the equipment.

The automated pump is set up to pump 15 mL +/- 1 mL per minute of solution from the cylinder to the specimen, after a 10 minute pre-pumping period to remove air and warm up the pump to ensure constant delivery. The needle end is supported by a ring stand fitted with a clamp for supporting the dispensing end during the test. The height of the needle is adjusted to provide a gap of 3 mm between the needle end and the body facing surface of the specimen. (If the specimen swells when wetted, the needle height should be adjusted during the test to maintain the 3 mm gap.)

To determine capacity, a specimen is placed onto the center of the sensing grid with the body side surface facing up, and is oriented to accept liquid at the point representing the intersection of the longitudinal and transverse centerlines. The pump and timer are activated, and the test concludes when liquid from the specimen migrates to the sensing grid and shuts off the pump and the timer. Absorbent capacity (mL per minute) is then calculated.

The artificial menses liquid used in the testing was made according to US Patent 5,883,231 from blood and egg white by separating the blood into plasma and red cells and separating the white into thick and thin portions, where "thick" means it has a viscosity after homogenization above about 20 centipoise at 150 sec⁻¹, combining the thick egg white with the plasma and thoroughly mixing, and finally adding the red cells and again thoroughly mixing. A more detailed procedure Follows:

Defibrinated swine blood, is separated by centrifuging at 3000 rpm for 30 minutes, though other methods or speeds and times may be used if effective. The plasma is separated and stored separately, the buffy coat removed and discarded and the packed red blood cells stored separately as well. It should be noted that the blood must be treated in some manner so that it may be processed without coagulating. Various methods are known to those skilled in the art, such as defibrinating the blood to remove the clotting fibrous materials, the addition or anti-coagulant chemicals and others. The blood must be non-coagulating in order to be useful and any method which accomplishes this without damaging the plasma and red cells is acceptable.

Jumbo chicken eggs are separated, the yolk and chalazae discarded and the egg white retained. The egg white is separated into thick and thin portions by straining the white through a 1000 micron nylon mesh for about 3 minutes, and the thinner portion discarded. The thick portion of egg white, which is retained on the mesh, is collected and drawn into a 60 cc (cm³) syringe, which is then placed on a programmable syringe pump and homogenized by expelling and refilling the contents five times. The amount of homogenization is controlled by the syringe pump rate of about 100 mL /min, and the tubing inside diameter of about 0.12 inches. After homogenizing the thick egg white has a viscosity of about 20 centipoise at 150 sec⁻¹ and is then placed in the centrifuge and spun to remove debris and air bubbles at about 3000 rpm for about 10 minutes.

After centrifuging, the thick, homogenized egg white, which contains ovamucin, is added to a 300 cc FENWAL® Transfer pack container using a syringe. Then 60 cc of the swine plasma is added to the FENWAL® Transfer pack container. The FENWAL® Transfer pack container is clamped, all air bubbles removed, and placed in a Stomacher lab blender where it is blended at normal (or medium) speed for about 2 minutes. The FENWAL® transfer pack container is then removed from the blender, 60 cc of swine red blood cells are added, and the contents mixed by hand kneading for about 2 minutes or until the contents appeared homogenous. A hematocrit of the final mixture should show a red blood cell content of about 30 weight percent and generally should be at least within a range of 28-32 weight percent for artificial menses made according to this Example. The amount of egg white is about 40 weight percent.

The ingredients and equipment used in the preparation of artificial menses are readily available. Below is a listing of sources for the items used, though of course other sources may be used providing they are approximately equivalent.

Blood (swine): Cocalico Biologicals, Inc., 449 Stevens Rd., Reamstown, PA 17567, (717) 336-1990.

Fenwal® Transfer pack container, 300 mL , with coupler, code 4R2014: Baxter Healthcare Corporation, Fenwal Division, Deerfield, IL 60015.

Harvard Apparatus Programmable Syringe Pump model no. 55-4143: Harvard Apparatus, South Natick, MA 01760.

Stomacher 400 laboratory blender model no. BA 7021, serial no. 31968: Seward Medical, London, England, UK.

1000 micron mesh, item no. CMN-1000-B: Small Parts, Inc., PO Box 4650, Miami Lakes, FL 33014-0650, 1-800-220-4242.

Hemata Stat-II device to measure hemocrits, serial no. 1194Z03127: Separation Technology, Inc., 1096 Rainer Drive, Altamont Springs, FL 32714.

Generally speaking, the thickness or caliper of the materials were measured at 0.05 psi (3.5 g/cm²) with a Starret-type bulk tester or other conventional bulk tester. The results of measurements are expressed in units of millimeters. The volumes reported for various components were calculated from measurements of length, width and thickness. These volumes were expressed in units of cubic centimeters (cm³).

As can be seen from the results, the pads were relatively even in terms of absorbency and absorbency capacity. However, the pads incorporating the multi-component liquid absorbent structure showed improved rewetting when compared to the commercial pads.

**TABLE 1**

| **METHOD / PRODUCT** | | | **MAXI PADS** | | | **SLIM PADS** | | |
|---|---|---|---|---|---|---|---|---|
| | | | | GEM II | Laurier | | GEM II | Laurier |
| Width (mm) | | Avg. | | 93.8 | 73.2 | | 93.4 | 73.4 |
| | | Std. | | 0.6 | 0.5 | | 0.7 | 0.7 |
| Length (mm) | | Avg. | | 230.0 | 219.8 | | 230.1 | 216.1 |
| | | Std. | | 0.7 | 0.8 | | 0.3 | 0.6 |
| Weight (gm) | | Avg. | | 11.8 | 8.8 | | 8.3 | 7.4 |
| | | Std. | | 0.4 | 0.1 | | 0.1 | 0.3 |
| Thickness (mm) STP 260-W | | Avg. | | 9.1 | 7.9 | | 8.3 | 6.5 |
| | | Std. | | 0.2 | 0.2 | | 0.3 | 0.5 |
| Absorbency Rate (sec.) STM 2447 | 10 mL. | Avg. | | 10.8 | 114. | | - | - |
| | Liquid | Std. | | 1.2 | 1.3 | | | |
| | 6 mL. | Avg. | | - | - | | 6.3 | 5.6 |
| | Liquid | Std. | | | | | 0.9 | 0.6 |
| Rewet test (gm) | | Avg. | | 0.3 | 1.4 | | 0.4 | 0.9 |
| STM.2440 Formerly | | | | 0.1 | 0.03 | | 0.1 | 0.04 |
| STP.682-W | | Std. | | | | | | |
| Absorbency capacity ( mL | | Avg. | | 105.2 | 135.8 | | 90.2 | 93.0 |
| .) | | | | 1.4 | 5.5 | | 6.6 | 4.2 |
| STM.2434 Formerly STP.191-W | | Std. | | | | | | |

### Example 2

In Example 2, two different ultra thin feminine pads were tested to determine the effectiveness of such absorbent articles containing a multi-component liquid absorbent structure when compared to a more conventional commercial feminine pad. In this Example, one pad including a multi-component liquid absorbent structure is identified as Goodfeel II, while the more conventional commercial feminine pad is a Whisper Ultra Thin Pad from Whisper (Thailand). Table 2 sets forth the specific measurements for each of the pads.

The Goodfeel II pad had a multi-component liquid absorbent structure in which the first liquid absorbent component had an area of about 38 square centimeters and the second liquid absorbent component had an area of about 131 square centimeters.

The first liquid absorbent component had a thickness of approximately 1.5 millimeters (0.15 cm) to yield a calculated volume of about 6 cm³. The Goodfeel II pad utilized a second liquid absorbent component formed of cellulose fluff. This component had a thickness of about 1.7 millimeters (0.17 cm) to yield a calculated volume of about 22 cm³.

The first liquid absorbent component was a material available from Buckeye Technologies, Inc. under the trade designation Unicore 8001. The material has a basis weight of approximately 230 gsm and a liquid absorbent capacity of approximately 6 grams of liquid per gram of material. The second liquid absorbent component was a batt of fluff pulp including about 0.25 gm of superabsorbent material. The second liquid absorbent component had a basis weight of approximately 213 gsm and a liquid absorbent capacity of approximately 10 grams of liquid per gram of material.

The control commercial feminine pad was a Whisper brand ultra-thin pad available in Thailand from the Procter & Gamble Company. Generally speaking, these pads have a liquid absorbent component that can be described as having a first non-woven surge / transfer layer supported on a spunbond or similar nonwoven layer. This layer had a total thickness of about 0.75 mm (0.075 cm) as well as a length of about 140 cm and a width of about 60 cm. The Whisper pad had a second absorbent layer composed of fluff pulp and superabsorbent material. This second layer had a thickness of about 1.5 mm (0.15 cm) and as well as a length of about 195 cm and a width of about 65 cm.

Each of the pads was tested for its absorbency rate using the testing procedure described above. Additionally, a rewet test was performed using the rewet testing procedure described above. Finally, each pad was tested for its absorbency capacity using absorbency capacity testing procedure described above. As can be seen from the results, the pads were relatively even in terms of absorbency capacity. However, the Goodfeel II pad absorbed liquid at a much faster rate and showed improved resistance to rewetting compared to the commercial pad.

**TABLE 2**

| **METHOD/PRODUCT** | | **GOOD FEEL II** | **WHISPER** |
|---|---|---|---|
| Width (mm) | Avg. | 84.9 | 90.8 |
| | Std. | 1.2 | |
| Length (mm) | Avg. | 235.5 | 226.2 |
| | Std. | 0.7 | |
| Weight (gm) | Avg. | 6.7 | 5.3 |
| | Std. | 0.1 | 0.1 |
| Thickness | Avg. | 3.8 | 2.8 |
| (mm) | Std. | 0.1 | |
| STP 260-W | | | |
| Absorbency rate (sec.) | Avg. | 8.0 | 24.7 |
| 6mLliquid | Std. | 1.5 | 2.2 |
| STM.2447 Formerly STP 89-W | | | |
| Rewet test (gm) | Avg. | 1.7 | 2.1 |
| STM.2440 Formerly | Std. | 0.2 | |
| STP 682-W | | | |
| Absorbency capacity ( mL | Avg. | 108.1 | 127.7 |
| ) | Std. | 1.3 | |
| STM 2434 Formerly STP 191-W | | | |

### Example 3

In Example 3, two different ultra thin feminine pads containing superabsorbent were tested to determine the effectiveness of such absorbent articles containing a multi-component liquid absorbent structure when compared to a more conventional commercial feminine pad. In this Example, one pad including a multi-component liquid absorbent structure is identified as C-Ultrathin, while the more conventional commercial feminine pad is a Whisper Ultra Thin Pad available in Hong Kong from the Procter & Gamble Company. Table 3 sets forth the specific measurements for each of the pads.

The C-Ultrathin pad had a Guial apertured film cover available from Guial Film of France and a liquid impermeable backsheet. The pad incorporated a multi-component liquid absorbent structure in which the first liquid absorbent component had an area of 38 square centimeters and the second liquid absorbent component had an area of about 131 square centimeters.

The first liquid absorbent component had a thickness of approximately 1.5 millimeters (0.15cm) to yield a calculated volume of 6 cm³.

The first liquid absorbent component was a material available from Buckeye Technologies under the trade designation Unicore 8001. The material has a basis weight of approximately 230 gsm and a liquid absorbent capacity of approximately 6 grams of liquid per gram of material. The second liquid absorbent component was a batt composed of about 60%, by weight, fluff pulp and about 40%, by weight, superabsorbent. The second liquid absorbent component has a basis weight of approximately 356 gsm and a liquid absorbent capacity of approximately 40 grams of liquid per gram of material.

The Whisper brand ultrathin commercial feminine pad also had an apertured film cover. Generally speaking, these pads have a liquid absorbent component that can be described as having a first air-laid layer supported on a spunbond or similar nonwoven layer. This layer had a total thickness of about 0.75 mm (0.075 cm) as well as a length of about 140 mm and a width of about 60 mm. The Whisper pad had a second absorbent layer composed of fluff pulp and superabsorbent material. This second layer was wrapped in thin airlaid material and had a thickness of about 1.5 mm (0.15 cm) and as well as a length of about 175 mm and a width of about 65 mm.

Each of the pads was tested for its absorbency, rewet characteristics and absorbency capacity using the testing procedures described above. As can be seen from the results, the pads were relatively even in terms of absorbency capacity. However, the C-Ultrathin pad absorbed liquid at a much faster rate and showed improved rewetting when compared to the commercial pad.

**TABLE 3**

| **METHOD/PRODUCT** | | **C-Ultrathin** | **WHISPER** |
|---|---|---|---|
| Width (mm) | Avg. | 145.4 | 155.2 |
| | Std. | 0.8 | |
| Length (mm) | Avg. | 261.3 | 221.4 |
| | Std. | 0.5 | |
| Weight (gm) | Avg. | 10.6 | 5.8 |
| | Std. | 0.2 | 0.0 |
| Thickness | Avg. | 3.8 | 3.0 |
| (mm) | Std. | 0.1 | |
| STP 260-W | | | |
| Absorbency rate (sec.) | Avg. | 7.9 | 24.3 |
| 6mLliquid | Std. | 0.3 | 1.6 |
| STM.2447 Formerly STP 89-W | | | |
| Rewet test (gm) | Avg. | 0.01 | 1.9 |
| STM.2440 Formerly | Std. | 0.01 | |
| STP 682-W | | | |
| Absorbency capacity ( mL | Avg. | 213.9 | 108.9 |
| ) | Std. | 4.5 | |
| STM 2434 Formerly STP 191-W | | | |
| Stain after | Avg. | 55.0 | 65.0 |
| Rewet Test | Std. | 0.5 | |
| Length (mm) | | | |
| Stain after | Avg. | 62.0 | 100.0 |
| Rewet Test | Std. | 1.0 | 0.7 |
| Width (mm) | | | |

### Example 4

This example illustrates how a relatively large proportion of an insult with an artificial menstrual liquid migrates quickly (after 1 minute) from the first liquid absorbent component to the second liquid absorbent component of a multi-component liquid absorbent structure.

The first liquid absorbent component is in the form of a coherent, flexible matrix including stratified layers of fibrous material. Two different types of materials were used for the first liquid absorbent component to illustrate that the certain types of multi-strata or stratified structures are useful.

The second liquid absorbent component is a batt composed of about 60%, by weight, fluff pulp and about 40%, by weight, superabsorbent. The second liquid absorbent component has a basis weight of approximately 356 gsm, a liquid absorbent capacity of approximately 16 grams of liquid per gram of material and an area of about 131 square centimeters.

One material used for the first liquid absorbent component was a material available from Buckeye Technologies under the trade designation Unicore 8001. The material has a basis weight of approximately 230 gsm and a liquid absorbent capacity of approximately 6 grams of liquid per gram of material. This material had an area of 38 square centimeters. The first liquid absorbent component had a thickness of approximately 1.5 millimeters (0.15cm) to yield a calculated volume of 6 cm³. This material is identified in Tables 4 and 5 as "Material #1".

Another material used for the first liquid absorbent component has basis weight of about 200 gsm. This material is identified in Tables 4 and 5 as "Material #2" and included a first air-laid layer having a basis weight of about 50 gsm. The first layer contained about 85%, by weight, polyester fibers - 15 denier per filament bonded together with about 15%, by weight, of a conventional latex binder suitable for personal care products. Material #2 included a second air-laid layer having a basis weight of about 150 gsm. This second layer contained about 90%, by weight, cellulose fluff and about 10%, by weight, bi-component binder fibers composed of a polyester core and a polyethylene or polyethylene-like sheath that softens or melts when heat is applied to dry the latex binder and thermally activate the binder fibers. This specific combination layers had an area of 38 square centimeters and a thickness of approximately 1.6 millimeters (0.16cm) to yield a calculated volume of about 6.1 cm³.

In each test, each component of the liquid absorbent structure was weighed and the weight was recorded. The structures were reassembled. An approximately 10 mL insult of artificial menses liquid was applied to the center portion of the first major surface of the first liquid absorbent component of the structures identified above. After about 60 seconds (1 minute) the components were separated and re-weighed. The difference between the wet weight and the dry weight was the amount of liquid retained by that component after the 60 second interval.

**TABLE 4**

| **Material** | **First component Weight** Dry | | **First component Weight** Wet | **Liquid Amount** |
|---|---|---|---|---|
| Material # 1 | Avg. | 0.87 | 2.97 | 2.10 |
| (grams) | Std. | 0.01 | 0.19 | |
| Material #2 | Avg. | 0.92 | 3.05 | 2.13 |
| (grams) | Std. | 0.00 | 0.12 | |

| **Material** | **Second component Weight** Dry | | **Second component Weight** Wet | **Liquid Amount** |
|---|---|---|---|---|
| Material # 1 | Avg. | 8.78 | 17.57 | |
| (grams) | Std. | 0.04 | 0.35 | |
| Material #2 | Avg. | 8.85 | 17.40 | |
| (grams) | Std. | 0.09 | 0.12 | |

| **Material** | **Cover Weight** Dry | | **Cover Weight** Wet | **Liquid Amount** |
|---|---|---|---|---|
| Material # 1 | Avg. | 0.69 | 0.71 | 0.02 |
| (grams) | Std. | 0.02 | 0.02 | 0.01 |
| Material #2 | Avg. | 0.69 | 0.71 | 0.02 |
| (grams) | Std. | 0.02 | 0.02 | 0.00 |

The amounts of liquid in each of the components after one minute were measured and a percent was calculated. The results are reported in Table 5.

**TABLE 5**

| **Material** | **Total Liquid Amount** | | | |
|---|---|---|---|---|
| Material # 1 | Avg. | 10.91 | | |
| (grams) | Std. | 0.46 | | |
| Material #2 | Avg. | 10.69 | | |
| (grams) | Std. | 0.05 | | |

| **Material** | **Percent in cover** | | **Percent in first component** | **Percent in second component** |
|---|---|---|---|---|
| Material # 1 | Avg. | 0.20 | 19.3 | 80 |
| | Std. | 0.07 | 1.44 | 1 |
| Material #2 | Avg. | 0.20 | 19.9 | 79 |
| | Std. | 0.04 | 1.09 | 1 |

While much of the description of the present invention has been directed to feminine pads, the present invention may also be used in other absorbent articles, such as diapers and incontinence articles. As such, the present invention has a wide array of utility and provides a substantial improvement over prior art absorbent articles in terms of protection, leakage and comfort to the wearer.

## Claims

1. A multi-component liquid absorbent structure comprising:
a first liquid absorbent component (118) comprising a coherent, flexible matrix, including stratified layers of fibrous material, the first liquid absorbent component having a first major surface, a second major surface, a first total area and a first liquid absorbent capacity, and
a second liquid absorbent component (116) comprising a layer of a different liquid absorbent material adjacent the second major surface of the first liquid absorbent component, the second liquid absorbent component having a second total area and a second liquid absorbent capacity,
wherein the first liquid absorbent component provides a distribution of interstitial spaces that are smaller in size adjacent the second major surface and larger in size adjacent the first major surface,
**characterised in that** the liquid absorbent structure is such that the ratio of the second total area to the first total area is greater than 3.5 to 1, the ratio of the second liquid absorbent capacity to the first liquid absorbent capacity is greater than 10 to 1 and an overall liquid absorbent capacity of the liquid absorbent structure is greater than about 35 grams, and **in that** the first liquid absorbent component provides a distribution of interstitial spaces that are smaller in size adjacent the second major surface than interstitial spaces in the second liquid absorbent component adjacent that second major surface.

2. The liquid absorbent structure of claim 1, the first liquid absorbent component having a first volume and the second liquid absorbent component having a second volume, the ratio of the second volume to the first volume is greater than about 10 to 1 and the total volume of the liquid absorbent structure is less than about 130 cm³.

3. The liquid absorbent structure of claim 1, the first liquid absorbent component having a first thickness and volume, and
the second liquid absorbent component having a second thickness and volume and a second liquid absorbent capacity,
wherein the thickness of first and second liquid absorbent component is each between 1 mm and 2 mm, the ratio of the second liquid absorbent capacity to the first liquid absorbent capacity is greater than 10; the ratio of the second volume to the first volume is greater than 10 to 1 and the total volume of the liquid absorbent structure is less than 30 cm³;
such that the first liquid absorbent component retains less than about 30% of an artificial menses liquid 1 minute after a 10 mL insult.

4. The liquid absorbent structure of claim 1 or 2, wherein the first liquid absorbent component retains less than 30% of an artificial menses liquid 1 minute after a 10 mL insult.

5. The liquid absorbent structure of claim 1 or 2, wherein the first liquid absorbent component retains less than 30% of an artificial menses liquid 1 minute after introduction of a 10 mL insult at the center of the structure.

6. The liquid absorbent structure of any preceding claim, wherein the first liquid absorbent component comprises strata of air-laid staple length fibers, air-laid fluff cellulose fibers, air-laid chemically modified cellulose fibers, hydrogel fibers and combinations thereof.

7. The liquid absorbent structure of claim 6, wherein the first liquid absorbent component further includes particulate materials.

8. The liquid absorbent structure of claim 6 or 7, wherein the first liquid absorbent component is bonded utilizing thermal binder fibers, adhesives, thermal point bonding, mechanical entanglement, latex emulsions and combinations thereof.

9. The absorbent structure of claim 6 or 7, wherein the first liquid absorbent component is bonded utilizing thermal binder fibers, adhesives, latex emulsions and combinations thereof.

10. The structure of any of claims 1 to 5, wherein the generally stratified layers of the first liquid absorbent component comprises at least two layers of a fibrous nonwoven web.

11. The structure of claim 10, wherein at least one of the layers of a fibrous nonwoven web is selected from bonded-carded webs, air-laid webs, meltblown fiber webs, spunbonded filament webs, hydraulically entangled fibrous webs, mechanically entangled fibrous webs and combinations thereof.

12. The liquid absorbent structure of any preceding claim, where in the second liquid absorbent component is selected from hydrogel containing composite structures, cellulose fluff structures, and generally homogeneous air-laid structures.

13. The multi-component liquid absorbent structure of any preceding claim, wherein the interstitial spaces at the second major surface of the first liquid absorbent component are configured to transfer liquid substantially along the length of the first liquid absorbent component in addition to releasing liquid to the second liquid absorbent component.

14. The multi-component liquid absorbent structure of any preceding claim, further comprising a channel (128) in the second liquid absorbent component spanning at least a portion of the periphery of the first liquid absorbent component.

15. The multi-component liquid absorbent structure of any preceding claim, wherein the first liquid absorbent component overlays at least about 50% of a stain area in the second liquid absorbent component generated by artificial menses liquid about 10 minutes after an approximately 5 mL insult introduced at the center of the first major surface of the first liquid absorbent component.

16. A sanitary napkin (110) having a longitudinal centerline and improved liquid management based on the utility of an absorbent multi-component structure, the sanitary napkin comprising:
a liquid permeable body-facing layer (112);
a liquid impermeable garment-facing layer (114); and
the multi-component absorbent structure of any preceding claim between the liquid permeable body-facing layer and the liquid impermeable garment-facing layer, wherein at least a portion of the first liquid component is positioned on the longitudinal centerline of the sanitary napkin.

## Patentansprüche

1. Flüssigkeitsabsorbierende Mehrkomponentenstruktur, umfassend:
eine erste flüssigkeitsabsorbierende Komponente (118), umfassend eine kohärente flexible Matrix, die geschichtete Lagen aus Faserstoff enthält, wobei die erste flüssigkeitsabsorbierende Komponente eine erste Hauptfläche, eine zweite Hauptfläche, eine erste Gesamtfläche und ein erstes Flüssigkeitsabsorptionsvermögen aufweist,
eine zweite flüssigkeitsabsorbierende Komponente (116), umfassend eine Lage aus einem verschiedenen flüssigkeitsabsorbierenden Stoff, angrenzend an die zweite Hauptfläche der ersten flüssigkeitsabsorbierenden Komponente, wobei die zweite flüssigkeitsabsorbierende Komponente eine zweite Gesamtfläche und ein zweites Flüssigkeitsabsorptionsvermögen aufweist,
wobei die erste flüssigkeitsabsorbierende Komponente eine Verteilung von interstitiellen Räumen bereitstellt, die angrenzend an die zweite Hauptfläche von kleinerer Größe sind und angrenzend an die erste Hauptfläche von größerer Größe sind,
und **dadurch gekennzeichnet, dass** die flüssigkeitsabsorbierende Struktur derartig ist, dass das Verhältnis der zweiten Gesamtfläche zu der ersten Gesamtfläche größer als 3,5 zu 1 ist, das Verhältnis des zweiten Flüssigkeitsabsorptionsvermögens zu dem ersten Flüssigkeitsabsorptionsvermögen 10 zu 1 ist und ein Gesamt-Flüssigkeitsabsorptionsvermögen der flüssigkeitsabsorbierenden Struktur größer als ungefähr 35 Gramm ist und dass die erste flüssigkeitsabsorbierende Komponente eine Verteilung von interstitiellen Räumen bereitstellt, die angrenzend an die zweite Hauptfläche von kleinerer Größe sind als interstitielle Räume in der zweiten flüssigkeitsabsorbierenden Komponente angrenzend an diese zweite Hauptfläche.

2. Flüssigkeitsabsorbierende Struktur nach Anspruch 1, wobei die erste flüssigkeitsabsorbierende Komponente ein erstes Volumen aufweist und die zweite flüssigkeitsabsorbierende Komponente ein zweites Volumen aufweist und wobei das Verhältnis des zweiten Volumens zu dem ersten Volumen größer als ungefähr 10 zu 1 ist und das Gesamtvolumen der flüssigkeitsabsorbierenden Struktur kleiner als ungefähr 130 m³ ist.

3. Flüssigkeitsabsorbierende Struktur nach Anspruch 1, wobei die erste flüssigkeitsabsorbierende Komponente eine erste Dicke und ein erstes Volumen aufweist und die zweite flüssigkeitsabsorbierende Komponente eine zweite Dicke, ein zweites Volumen und ein zweites Flüssigkeitsabsorptionsvermögen aufweist,
wobei die Dicke der ersten und der zweiten flüssigkeitsabsorbierenden Komponente jeweils zwischen 1 mm und 2 mm ist, das Verhältnis des zweiten Flüssigkeitsabsorptionsvermögens zu dem ersten Flüssigkeitsabsorptionsvermögen größer als 10 ist, das Verhältnis des zweiten Volumens zu dem ersten Volumen größer als 10 zu 1 ist und das Gesamtvolumen der flüssigkeitsabsorbierenden Struktur kleiner als ungefähr 30 m³ ist,
so dass die erste flüssigkeitsabsorbierende Komponente weniger als ungefähr 30% einer künstlichen Menstruationsflüssigkeit nach Angriff von 10 Milliliter für 1 Minute hält.

4. Flüssigkeitsabsorbierende Struktur nach Anspruch 1 oder 2, wobei die erste flüssigkeitsabsorbierende Komponente weniger als ungefähr 30% einer künstlichen Menstruationsflüssigkeit nach Angriff von 10 Milliliter für 1 Minute hält.

5. Flüssigkeitsabsorbierende Struktur nach Anspruch 1 oder 2, wobei die erste flüssigkeitsabsorbierende Komponente weniger als ungefähr 30% einer künstlichen Menstruationsflüssigkeit, nach dem Einfließen eines Angriffs von 10 Milliliter auf der Mitte der Struktur, für 1 Minute hält.

6. Flüssigkeitsabsorbierende Struktur nach einem der vorhergehenden Ansprüche,
wobei die erste flüssigkeitsabsorbierende Komponente Schichten aus Airlaid-Kurzfasern, Airlaid-Zellulose-Fluff-Fasern, chemisch modifizierten Airlaid-Zellulosefasern, Hydrogelfasern und Kombinationen davon umfasst.

7. Flüssigkeitsabsorbierende Struktur nach Anspruch 6, wobei die erste flüssigkeitsabsorbierende Komponente des Weiteren Partikelmaterial enthält.

8. Flüssigkeitsabsorbierende Struktur nach Anspruch 6 oder 7, wobei die erste flüssigkeitsabsorbierende Komponente unter Verwendung von thermischen Binderfasern, Klebstoffen, thermischem Point-Bonding, mechanischer Vernadelung, Latexemulsionen und Kombinationen davon gebondet wird.

9. Flüssigkeitsabsorbierende Struktur nach Anspruch 6 oder 7, wobei die erste flüssigkeitsabsorbierende Komponente unter Verwendung von thermischen Binderfasern, Klebstoffen, Latexemulsionen und Kombinationen davon gebondet wird.

10. Struktur nach einem der Ansprüche 1 bis 5, wobei die generell geschichteten Lagen der ersten flüssigkeitsabsorbierenden Komponente wenigstens zwei Lagen aus einem Faservliesgewebe enthalten.

11. Struktur nach Anspruch 10, wobei wenigstens eine der Lagen aus einem Faservliesgewebe aus gebondeten kardierten Geweben, Airlaid-Geweben, schmelzgesponnenen Fasergeweben, Spinnvlies-Filamentgeweben, wasservernadelten Fasergeweben, mechanisch vernadelten Fasergeweben und Kombinationen davon ausgewählt wird.

12. Flüssigkeitsabsorbierende Struktur nach einem der vorhergehenden Ansprüche,
wobei die zweite flüssigkeitsabsorbierende Komponente aus einem Verbundstrukturen enthaltenden Hydrogel, Zellulose-Fluff-Strukturen und generell homogenen Airlaid-Strukturen ausgewählt wird.

13. Flüssigkeitsabsorbierende Mehrkomponentenstruktur nach einem der vorhergehenden Ansprüche, wobei die interstitiellen Räume an der zweiten Hauptfläche der ersten flüssigkeitsabsorbierenden Komponente konfiguriert sind, um, zusätzlich zu dem Abgeben von Flüssigkeit zu der zweiten flüssigkeitsabsorbierenden Komponente, Flüssigkeit im Wesentlichen entlang der Länge der ersten flüssigkeitsabsorbierenden Komponente weiterzuleiten.

14. Flüssigkeitsabsorbierende Mehrkomponentenstruktur nach einem der vorhergehenden Ansprüche, des Weiteren einen Kanal (128) in der zweiten flüssigkeitsabsorbierenden Komponente umfassend, der sich wenigstens über einen Teil der Peripherie der ersten flüssigkeitsabsorbierenden Komponente erstreckt.

15. Flüssigkeitsabsorbierende Mehrkomponentenstruktur nach einem der vorhergehenden Ansprüche, wobei die erste flüssigkeitsabsorbierende Komponente wenigstens ungefähr 50% eines Schmutzbereiches in der zweiten flüssigkeitsabsorbierenden Komponente überlagert, der durch künstliche Menstruationsflüssigkeit ungefähr 10 Minuten nach einem Angriff von ungefähr 5 Milliliter, eingeflossen in der Mitte der ersten Hauptfläche der ersten flüssigkeitsabsorbierenden Komponente, erzeugt wurde.

16. Damenbinde (110) mit einer Längsmittellinie und verbesserter Flüssigkeitsverwaltung auf Basis der Nutzung einer flüssigkeitsabsorbierenden Mehrkomponentenstruktur, wobei die Damenbinde umfasst:
eine flüssigkeitsdurchlässige körperseitige Lage (112),
eine flüssigkeitsundurchlässige wäscheseitige Lage (114) und
die flüssigkeitsabsorbierende Mehrkomponentenstruktur nach einem der vorhergehenden Ansprüche zwischen der flüssigkeitsdurchlässigen körperseitigen Lage und der flüssigkeitsundurchlässigen wäscheseitigen Lage, wobei wenigstens ein Teil der ersten flüssigkeitsabsorbierenden Komponente auf der Längsmittellinie der Damenbinde angeordnet ist.

## Revendications

1. Structure absorbant les liquides à composants multiples comprenant :
un premier composant absorbant les liquides (118) comprenant une matrice cohérente flexible, incluant des couches stratifiées de matière fibreuse, le premier composant absorbant les liquides présentant une première surface principale, une deuxième surface principale, une première surface totale et une première capacité d'absorption des liquides, et
un deuxième composant absorbant les liquides (116) comprenant une couche d'une matière absorbant les liquides différente adjacente à la deuxième surface principale du premier composant absorbant les liquides, le deuxième composant absorbant les liquides présentant une deuxième surface totale et une deuxième capacité d'absorption des liquides,
dans laquelle le premier composant absorbant les liquides assure une distribution telle que les espaces interstitiels sont plus petits à proximité de la deuxième surface principale et plus grands à proximité de la première surface principale,
**caractérisée en ce que** la structure absorbant les liquides est telle que le rapport de la deuxième surface totale sur la première surface totale est supérieur à 3,5:1, le rapport de la deuxième capacité d'absorption des liquides sur la première capacité d'absorption des liquides est supérieur à 10:1 et la capacité d'absorption des liquides globale de la structure absorbant les liquides est supérieure à environ 35 grammes, et **en ce que** le premier composant absorbant les liquides assure une distribution telle que les espaces interstitiels sont plus petits à proximité de la deuxième surface principale que les espaces interstitiels dans le deuxième composant absorbant les liquides à proximité de cette deuxième surface principale.

2. Structure absorbant les liquides selon la revendication 1, le premier composant absorbant les liquides présentant un premier volume et le deuxième composant absorbant les liquides présentant un deuxième volume, le rapport du deuxième volume sur le premier volume étant supérieur à environ 10:1 et le volume total de la structure absorbant les liquides étant inférieur à environ 130 cm³.

3. Structure absorbant les liquides selon la revendication 1, le premier composant absorbant les liquides présentant une première épaisseur et un premier volume et
le deuxième composant absorbant les liquides présentant une deuxième épaisseur et un deuxième volume et une deuxième capacité d'absorption des liquides,
l'épaisseur du premier et du deuxième composant absorbant les liquides étant comprise entre 1 mm et 2 mm, le rapport de la deuxième capacité d'absorption des liquides sur la première capacité d'absorption des liquides étant supérieur à 10 ; le rapport du deuxième volume sur le premier volume étant supérieur à 10:1 et le volume total de la structure absorbant les liquides étant inférieur à 30 cm³ ;
de telle sorte que le premier composant absorbant les liquides retient moins d'environ 30 % d'un liquide menstruel artificiel 1 minute après une charge de 10 ml.

4. Structure absorbant les liquides selon la revendication 1 ou 2, dans laquelle le premier composant absorbant les liquides retient moins de 30 % d'un liquide menstruel artificiel 1 minute après une charge de 10 ml.

5. Structure absorbant les liquides selon la revendication 1 ou 2, dans laquelle le premier composant absorbant les liquides retient moins de 30 % d'un liquide menstruel artificiel 1 minute après l'introduction d'une charge de 10 ml au centre de la structure.

6. Structure absorbant les liquides selon l'une quelconque des revendications précédentes, dans laquelle le premier composant absorbant les liquides comprend des strates de fibres courtes air-laid, de fibres de fluff de cellulose air-laid, de fibres de cellulose chimiquement modifiées air-laid, de fibres d'hydrogel et des combinaisons de celles-ci.

7. Structure absorbant les liquides selon la revendication 6, dans laquelle le premier composant absorbant les liquides inclut en outre des matières particulaires.

8. Structure absorbant les liquides selon la revendication 6 ou 7, dans laquelle le premier composant absorbant les liquides est lié en utilisant des fibres thermoliantes, des adhésifs, un liage thermique par points, un enchevêtrement mécanique, des émulsions de latex et des combinaisons de ceux-ci.

9. Structure absorbante selon la revendication 6 ou 7, dans laquelle le premier composant absorbant les liquides est lié en utilisant des fibres thermoliantes, des adhésifs, des émulsions de latex et des combinaisons de ceux-ci.

10. Structure selon l'une quelconque des revendications 1 à 5, dans laquelle les couches généralement stratifiées du premier composant absorbant les liquides comprennent au moins deux couches d'une nappe fibreuse non tissée.

11. Structure selon la revendication 10, dans laquelle au moins l'une des couches d'une nappe fibreuse non tissée est choisie parmi les nappes cardées liées, les nappes air-laid, les nappes de fibres obtenues par fusion-soufflage, les nappes à filaments filés-liés, les nappes fibreuses à enchevêtrement hydraulique, les nappes fibreuses à enchevêtrement mécanique et des combinaisons de celles-ci.

12. Structure absorbant les liquides selon l'une quelconque des revendications précédentes, dans laquelle le deuxième composant absorbant les liquides est choisi parmi des structures composites contenant de l'hydrogel, des structures de fluff de cellulose et des structures air-laid généralement homogènes.

13. Structure absorbant les liquides à composants multiples selon l'une quelconque des revendications précédentes, dans laquelle les espaces interstitiels au niveau de la deuxième surface principale du premier composant absorbant les liquides sont configurés de manière à transférer un liquide sensiblement sur la longueur du premier composant absorbant les liquides, en plus de dégager le liquide vers le deuxième composant absorbant les liquides.

14. Structure absorbant les liquides à composants multiples selon l'une quelconque des revendications précédentes, comprenant en outre un canal (128) dans le deuxième composant absorbant les liquides couvrant au moins une partie de la périphérie du premier composant absorbant les liquides.

15. Structure absorbant les liquides à composants multiples selon l'une quelconque des revendications précédentes, dans laquelle le premier composant absorbant les liquides recouvre au moins environ 50 % d'une surface de tâche dans le deuxième composant absorbant les liquides générée par un liquide menstruel artificiel environ 10 minutes après l'introduction d'une charge d'environ 5 ml au centre de la première surface principale du premier composant absorbant les liquides.

16. Serviette hygiénique (110) présentant un axe longitudinal et une gestion des liquides améliorée basée sur l'utilité d'une structure absorbante à composants multiples, la serviette hygiénique comprenant :
une couche côté corps (112) perméable aux liquides ;
une couche côté vêtement (114) imperméable aux liquides ; et
la structure absorbante à composants multiples selon l'une quelconque des revendications précédentes entre la couche côté corps perméable aux liquides et la couche côté vêtement imperméable aux liquides, dans laquelle au moins une partie du premier composant liquide est positionnée sur l'axe longitudinal de la serviette hygiénique.
